# EUROPÄISCHE PATENTANMELDUNG

(11) **EP 1 915 982 A1**
(43) Veröffentlichungstag der Anmeldung: **30.04.2008**
(21) Anmeldenummer: 06122665.0
(22) Anmeldetag: 20.10.2006
(51) Int. Cl.: A61K 8/34, A61Q 19/00

(54) **Verwendung von 1,2-Decandiol zur Sebumreduktion bzw. zur Unterstützung des Eindringens von Wirkstoffen in Hautbereiche, sowie kosmetische und/oder dermatologische Zubereitungen umfassend 1,2-Decandiol**

(71) Anmelder: Symrise GmbH & Co. KG, 37603 Holzminden (DE)
(72) Erfinder: Schmaus, Gerhard, 37671, Höxter (DE); Lange, Sabine, 37603, Holzminden (DE); Röding, Joachim, 79410, Badenweiler (DE)
(74) Vertreter: Eisenführ, Speiser & Partner

(57) **Zusammenfassung**

Die vorliegende Erfindung betrifft eine Verwendung von 1,2-Decandiol zur Reduktion der Sebumkonzentration der Haut, topisch zu applizierende, kosmetische und/oder dermatologische Zubereitung umfassend 1,2-Decandiol, ein Verfahren zur Reduktion der Sebumkonzentration der Haut sowie eine Verwendung von 1,2-Decandiol und ein Verfahren zur Unterstützung des Eindringens eines, zweier, dreier, vierer oder mehrerer Wirkstoffe in Hautbereiche mit gesteigerter Sebumproduktion.

## Beschreibung

Die vorliegende Erfindung betrifft eine Verwendung von 1,2-Decandiol zur Reduktion der Sebumkonzentration der Haut, topisch zu applizierende, kosmetische und/oder dermatologische Zubereitung umfassend 1,2-Decandiol, ein Verfahren zur Reduktion der Sebumkonzentration der Haut sowie eine Verwendung von 1,2-Decandiol und ein Verfahren zur Unterstützung des Eindringens eines, zweier, dreier, vierer oder mehrerer Wirkstoffe in Hautbereiche mit gesteigerter Sebumproduktion.

Seborrhoe (auch genannt Seborrhoea oder Schmerfluss) steht für die Überproduktion von Sebum (Talg) in einer Talgdrüse. Die Überproduktion an Sebum verursacht, dass die Talgdrüsenfollikel mit Sebum teilweise oder vollständig angefüllt sind. Dieses Sebum weist oftmals eine sehr feste Konsistenz und demzufolge eine niedrige Spreitung auf, so dass es nur sehr schwierig aus dem Talgdrüsenfollikel freigesetzt wird und sich auf der Haut ausbreiten kann. In Folge dessen reichert sich das Sebum in unerwünscht hohem Maße in den Talgdrüsen an und bildet einen idealen Nährboden für Mikroorganismen, wie insbesondere *Propionibacterium acnes*, einem bei der Entstehung von Akne maßgeblich beteiligten Mikroorganismus.

Bei fettig-öliger Haut führt die Überproduktion von Sebum zu einem unerwünschten Fettglanz auf der Haut. Im Bereich einer fettig-öligen Kopfhaut führt die Überproduktion von Sebum zu einem unerwünschten Fettglanz des Ansatzes der Haare.

Bei unreiner Haut führt die Überproduktion an Sebum zu Hautunebenheiten, wie z.B. Pickeln und Pusteln. Ein krankhaft verändertes Hautbild der unreinen Haut ist die Akne.

Bei Akne, die gewöhnlich in Pubertätsalter auftritt, ist der anaerobe Mikroorganismus *Propionibacterium acnes (P. acnes)* maßgeblich an der Entstehung beteiligt und zersetzt das Sebum zu Glycerin und Fettsäuren, wodurch die Talgdrüsen wiederum angeregt werden vermehrt Sebum herzustellen und wobei diese Abbauprodukte die Follikelwandungen angreifen beziehungsweise zerstören. Dies ruft regelmäßig Entzündungen in der Haut (Pickel, Pusteln, Knoten, Zysten) hervor, welche oft nur narbig ausheilen, wodurch das optische Erscheinungsbild des an unreiner Haut leidenden Menschen dauerhaft geschädigt wird (W. Umbach [Hrsg], Kosmetik, Entwicklung, Herstellung und Anwendung kosmetischer Mittel, 2.Aufl. Thieme Verlag, Stuttgart, 1995).

Herkömmliche Produkte zur Behandlung von fettiger und unreiner Haut, wie z.B. wässrig-ethanolische und/oder Tensid-haltige Reinigungsprodukte haben in der Regel den Nachteil die Haut zu strapazieren, auszutrocknen und wenig pflegend zu wirken. Besonders nachteilig ist, dass der Körper auf die Verwendung ethanolischer und/oder Detergentien-haltiger Lösungen über einen längeren Zeitraum hinweg mit einer Überproduktion von Sebum reagieren kann, was dem vorrangigen therapeutischen Ziel der Sebumreduktion bei fettig-öliger, unreiner und zu Akne neigender Haut genau entgegenwirkt.

Zur topischen Behandlung der Akne stehen Aknetherapeutika, wie zum Beispiel starke Oxidationsmittel, wie z.B Benzoylperoxid; alpha-Hydroxysäuren, wie z.B. Salicylsäure und Milchsäure; aliphatische Dicarbonsäuren, wie z.B. Azelainsäure; Retinoide, wie z.B. Tretionoin (Synonym: all-trans-Retinsäure), all-trans-Retinal und cis-13-Retinsäure (Isotretinoin); Antiandrogene (5alpha-Reductasehemmer) sowie Antibiotika, wie z.B. Clindamycin, Tetracyclin und Erythromycin zur Verfügung. Darüber hinaus kann eine abrasive Behandlung durchgeführt werden. Entsprechende Präparate enthalten Aluminiumoxid-Partikel oder Silikonharz.

Die Wirksamkeit der starken Oxidationsmittel, wie z.B Benzoylperoxid; der alpha-Hydroxysäuren, wie z.B. Salicylsäure und Milchsäure sowie der aliphatischen Dicarbonsäuren, wie z.B. Azelainsäure wird dabei u.a. auf die Inhibition von *P*. *acnes* zurückgeführt. Die genannten Stoffe besitzen jedoch zumeist nur eine sehr moderate antimikrobielle Wirksamkeit gegenüber *P*. *acnes* und müssen daher in verhältnismäßig hoher Konzentration - Benzoylperoxi d z.B. mit bis zu 5 Gew.-% und Azelainsäure mit bis zu 20 Gew.-% - in kosmetischen und dermatologischen Formulierungen eingesetzt werden. Bedingt durch die hohe Dosierung wird jedoch äußerst nachteilig die Haut sehr stark strapaziert, was sich insbesondere in starker Austrocknung der Haut äußert, die zum Teil aufgrund der starken Reduktion des pH-Wertes der Haut häufig mit starken Hautirritationen einhergeht.

Retinoide besitzen ein breites Wirksamkeitsspektrum. Die Wirkung beruht dabei insbesondere auf der Normalisierung der mit Akne einhergehenden follikulären Hyperkeratiose, der Auflösung von Komedonen und auf einer Inhibition der Sebumproduktion. Nachteilig ist jedoch, dass die meisten Retinoide ebenfalls stark irritierende Nebenwirkungen haben. Isotretinoin zeigt darüber hinaus eine teratogene Wirksamkeit und kann daher nicht zur Behandlung stark fettiger, unreiner und zu Akne neigender Haut von Schwangeren eingesetzt werden.

Die topisch sowie systemisch anzuwendenden Antibiotika, wie z.B. Clindamycin, Erythromycin und Tetracyclin besitzen eine stark antimikrobielle Wirksamkeit gegenüber *P*. *acnes,* wobei sie MHK-Werte im Bereich < 1 pmm aufweisen. Insbesondere aufgrund des in der klinischen Praxis immer häufiger zu beobachtenden Auftretens resistenter *P*. *acnes* Stämme sowie deren unspezifischem Wirkungsspektrum, welches zu einer starken Beeinträchtigung der gesunden humanen Mikroflora führt, geraten auch die in der Akne-Therapie geläufigen Antibiotika zunehmend ins Licht der Kritik.

Zur Behandlung von Akne wird in EP 1 598 064 A1 eine Wirkstoffskombination aus alpha- und/oder beta-Hydroxysäuren und Alkan-1,2-Diolen vorgeschlagen, wobei beschrieben wird, dass die Kombination von 1 % Milchsäure mit 0,2 % 1,2-Decandiol eine verbesserte antibakterielle Wirksamkeit der Kombination in Bezug auf *P*. *acnes* gegenüber des Einzelbestandteilen, insbesondere von 0,2 % 1,2-Decandiol aufweist. Bei der alleinigen Gabe von 0,2 % 1,2-Decandiol ist so gut wie keine antibakterielle Wirkung im Suspensionstest ersichtlich.

Eine Aufgabe der vorliegenden Erfindung besteht nun darin, eine Verbindung und/oder eine Zubereitung zur Reduktion der Sebumkonzentration in der Haut bereitzustellen, um insbesondere bei fettig-öliger Haut den Fettglanz zu reduzieren und/oder zu verhindern und/oder bei unreiner Haut, die sich insbesondere durch Hautunebenheiten, wie z.B. Pickel und Pusteln auszeichnet, eine Verminderung der Hautunebenheiten zu erreichen.

Zusätzlich soll die Verbindung und/oder die Zubereitung pflegend wirken und zudem die Haut und/oder Haare nicht strapazieren und/oder austrocknen.

Die vorstehenden Aufgaben werden vollständig oder zumindest zum Teil durch die Gegenstände der vorliegenden Erfindung, wie sie insbesondere in den Ansprüchen wiedergegeben sind, gelöst.

Eine Ausgestaltung der vorliegenden Erfindung betrifft eine Verwendung von 1,2-Decandiol zur Reduktion der Sebumkonzentration der Haut.

Eine weitere Ausgestaltung der vorliegenden Erfindung betrifft eine Verwendung von 1,2-Decandiol zur Herstellung eines topisch zu applizierenden, kosmetischen und/oder dermatologischen Mittels zur Reduktion der Sebumkonzentration der Haut.

Eine weitere Ausgestaltung der vorliegenden Erfindung betrifft eine topisch zu applizierende, kosmetische und/oder dermatologische Zubereitung dadurch gekennzeichnet, dass die Zubereitung folgende Bestandteile umfasst oder aus diesen Bestandteile besteht:
- eine Menge an 1,2-Decandiol die ausreicht, um die Sebumkonzentration der Haut zu reduzieren,
- gegebenenfalls ein, zwei, drei, vier, fünf, sechs, sieben, acht, neun, zehn oder mehrere Reinigungshilfsstoffe,
- gegebenenfalls ein, zwei, drei, vier oder mehrere Wirkstoffe ausgewählt aus der Gruppe bestehend aus Benzoylperoxid; Salicylsäure; Milchsäure; Azelainsäure; Retinoide, bevorzugt all-trans-Retinsäure, all-trans-Retinal, cis-13-Retinsäure und Adaptalen; Antibiotika, bevorzugt Clindamycin, Erythromycin und Tetracyclin ; Schwefel; Chlorhexidin; Triclosan; Farnesol; Phenoxyethanol; Isoflavonoide, bevorzugt Genistein, Daidzein, Genistin und Daizin und Antiandrogene, bevorzugt 5aplha-Reduktasehemmer
und
- gegebenenfalls ein oder mehrere weitere Hilfs- und/oder Zusatzstoffe mit der Maßgabe, dass wenn die Zubereitung eine Konzentration von 1 Gew.-% Milchsäure enthält, die Konzentration von 1,2-Decandiol nicht 0,2 Gew.-% betragen darf, jeweils bezogen auf das Gesamtgewicht der Zubereitung.

Eine weitere Ausgestaltung der vorliegenden Erfindung betrifft ein Verfahren zur Reduktion der Sebumkonzentration der Haut, dadurch gekennzeichnet, dass das Verfahren folgende Schritte umfasst oder aus folgenden Schritten besteht:
a) Bereitstellung von 1,2-Decandiol;
b) Herstellung einer erfindungsgemäßen Zubereitung, wie vorstehend beschrieben und
c) Applikation der nach b) hergestellten Zubereitung auf fettig-ölige und/oder unreine Haut, wobei die Menge an 1,2-Decandiol ausreicht, um die Sebumkonzentration der Haut zu reduzieren.

Eine weitere Ausgestaltung der vorliegenden Erfindung betrifft eine Verwendung von 1,2-Decandiol zur Unterstützung des Eindringens eines, zweier, dreier oder mehrerer Wirkstoffe ausgewählt aus der Gruppe bestehend aus Benzoylperoxid, Salicylsäure, Milchsäure, Azelainsäure, Retinoide, Antibiotika, Schwefel, Chlorhexidin, Triclosan, Farnesol, Phenoxyethanol, Isoflavonoide und Antiandrogene in Hautbereiche mit gesteigerter Sebumproduktion.

Eine weitere Ausgestaltung der vorliegenden Erfindung betrifft ein Verfahren zur Unterstützung des Eindringens eines, zweier, dreier oder mehrerer Wirkstoffe ausgewählt aus der Gruppe bestehend aus Benzoylperoxid, Salicylsäure, Milchsäure, Azelainsäure, Retinoide, Antibiotika, Schwefel, Chlorhexidin, Triclosan, Farnesol, Phenoxyethanol, Isoflavonoide und Antiandrogene in Hautbereiche mit gesteigerter Sebumproduktion, dadurch gekennzeichnet, dass das Verfahren folgende Schritte umfasst oder aus folgenden Schritten besteht:
a) Bereitstellung von 1,2-Decandiol,
b) Bereitstellung eines, zweier, dreier oder mehrerer Wirkstoffe ausgewählt aus der Gruppe bestehend aus Benzoylperoxid, Salicylsäure, Milchsäure, Azelainsäure, Retinoide, Antibiotika, Schwefel, Chlorhexidin, Triclosan, Farnesol, Phenoxyethanol, Isoflavonoide, Antiandrogene,
c) Herstellung einer topisch zu applizierenden, kosmetischen und/oder dermatologischen Zubereitung umfassend (oder bestehend aus)
   - eine Menge an 1,2-Decandiol die ausreicht, um die Sebumkonzentration der Haut zu reduzieren,
   - eine wirksame Menge eines, zweier, dreier, vierer oder mehrerer Wirkstoffe ausgewählt aus der Gruppe bestehend aus Benzoylperoxid, Salicylsäure, Milchsäure, Azelainsäure, Retinoide, Antibiotika, Schwefel, Chlorhexidin, Triclosan, Farnesol, Phenoxyethanol, Isoflavonoide und Antiandrogene
   - gegebenenfalls ein, zwei, drei, vier, fünf, sechs, sieben, acht, neun, zehn oder mehrere Reinigungshilfsstoffe und
   - gegebenenfalls ein oder mehrere weitere Hilfs- und/oder Zusatzstoffe mit der Maßgabe, dass wenn die Zubereitung eine Konzentration von 1 Gew.-% Milchsäure enthält, die Konzentration von 1,2-Decandiol nicht 0,2 Gew.-% betragen darf, jeweils bezogen auf das Gesamtgewicht der Zubereitung und
d) Applikation der nach c) hergestellten Zubereitung auf relevante Hautbereiche.

Es sei darauf hingewiesen, dass der Begriff 1,2-Decandiol im Rahmen der vorliegenden erfindungsgemäßen Ausgestaltungen sowohl (a) das 2S-konfigurierte Enantiomer als auch (b) das 2R-konfigurierte Enantiomer sowie (c) beliebige Mischungen aus 2S- und 2R-konfigurierten Decandiolen umfasst. Aus kommerziellen Gründen ist es zwar besonders vorteilhaft, das Razemat des 1,2-Decandiols zur Behandlung von fettiger-öliger und unreiner Haut sowie zur Prophylaxe und/oder Behandlung von Seborrhoe, insbesondere Akne, einzusetzen, da diese synthetisch besonders leicht zugänglich sind, die reinen Enantiomere oder nicht-razemische Mischungen dieser Enantiomere sind aber ebenfalls für die erfindungsgemäßen Zwecke geeignet.

Die vorstehenden Ausgestaltungen der vorliegenden Erfindung beruhen auf der Erkenntnis, dass 1,2-Decandiol den Tropfpunkt und damit die Fluidität von Sebum signifikant reduziert. Diese Erniedrigung des Tropfpunktes von Sebum ist mit Hilfe einer in vitro Studie an einem Modellsebum (siehe Beispiel 1) nachgewiesen worden. Diese spezifische Eigenschaft des 1,2-Decandiols führt dazu, dass durch die erhöhte Fluidität des Sebums die Spreitungsfähigkeit des Sebums erhöht wird und so das Sebum eine erhöhte Fähigkeit besitzt sich aus den Talgdrüsenfollikeln heraus auf der Haut auszubreiten.

Anhand klinischer *in vivo* Studien (Beispiel 2) wurde gezeigt, dass durch Einsatz von 1,2-Decandiol die Sebumkonzentration der Haut von jugendlichen Probanden im Alter von 17 - 19 Jahren mit fettig-öliger und/oder unreiner, insbesondere zu Akne neigender Haut signifikant reduziert werden konnte.

Im Sinne der vorliegenden Erfindung ist unter dem Begriff "Haut" menschliche oder tierische Haut gemeint.

Unter "Reduktion der Sebumkonzentration der Haut" ist in der vorliegenden Erfindung eine signifikante Reduktion der Sebumkonzentration der Haut zu verstehen, wobei eine Reduktion der Sebumkonzentration in der Haut, insbesondere in den Talgdrüsenfollikeln, und/oder auf der Haut und/oder bei einer behaarten Kopfhaut, auf den Haaren umfasst ist. Diese Reduktion der Sebumkonzentration der Haut kann beispielsweise mittels Sebumetrie, insbesondere mittels einem Sebumeter®SM 810, erhältlich von Courage & Khazaka, Köln, Deutschland, ermittelt werden.

Die Ursache hierfür ist noch nicht vollständig geklärt, ist aber wohl auf eine durch Fluidisierung hervorgerufene verbesserte Löslichkeit des Sebums und eine damit verbundene verbesserte mechanische Abtragung des Sebums zurückzuführen. Aufgrund des amphiphilen, lösungsvermittelnden Charakters von 1,2-Decandiol können dabei auch tensid-ähnliche Effekte des 1,2-Decandiols zur verbesserten Abtragung des hochlipophilen Sebums beitragen.

Folglich führt die Verwendung von 1,2-Decandiol zu einer Reduktion der Sebumkonzentration der Haut, wodurch Fettglanz bei fettig-öliger Haut und/oder Hautunebenheiten bei unreiner Haut verringert werden können.

Zudem wird angenommen, dass die Reduktion der Sebumkonzentration der Haut, insbesondere in den Talgdrüsenfollikeln, aber auch auf der Oberfläche der Haut, das Eindringen eines, zweier, dreier, vierer oder mehrerer Wirkstoffe ausgewählt aus der Gruppe bestehend aus Benzoylperoxid; Salicylsäure; Milchsäure; Azelainsäure; Retinoide, bevorzugt all-trans-Retinsäure, all-trans-Retinal, cis-13-Retinsäure und Adaptalen; Antibiotika, bevorzugt Clindamycin, Erythromycin und Tetracyclin; Schwefel; Chlorhexidin; Triclosan; Farnesol; Phenoxyethanol; Isoflavonoide, bevorzugt Genistein, Daidzein, Genistin und Daizin und Antiandrogene, bevorzugt 5aplha-Reduktasehemmer in Hautbereiche mit gesteigerter Sebumproduktion unterstützt. Es wird angenommen, dass durch die Reduktion des Sebums die Oberfläche der Haut dem oder den jeweiligen Wirkstoffen in Größerem Ausmaß für die jeweilige Wirkung zur Verfügung steht.

Hautbereiche mit gesteigerter Sebumproduktion zeichnen sich durch eine erhöhte Anzahl an und/oder Aktivität der Talgdrüsenfollikeln aus und sind bevorzugt im Gesichtsbereich, weiter bevorzugt Stirn, Nase, Wange, Kinn, behaarte Kopfhaut; vordere und hintere Schweißrinne; Nacken; Brust und Rücken zu finden. Weiter bevorzugt sind die Bereiche Stirn, Nase, Wange, Kinn, behaarte Kopfhaut, Nacken, Brust und Rücken.

Die antimikrobielle Wirkung aliphatischer Alkan-1,2-diole ist an sich bereits hinlänglich bekannt. Allerdings wird in keinem der Dokumente des Standes der Technik beschrieben, dass 1,2-Decandiol eine Reduktion der Sebumkonzentration in der Haut verursacht.

WO 03/000220 (Dragoco Gerberding & Co. KG) beschreibt die Verwendung von 1,2-Decandiol lediglich gegen Körpergeruch verursachende Bakterien. Eine Reduktion der Sebumkonzentration der Haut durch Verwendung von 1,2- Decandiol wird dort nicht offenbart.

Eine Reihe weiterer Dokumente befasst sich entweder mit 1,2-Decandiol, ohne dabei die Reduktion der Sebumkonzentration der Haut durch Verwendung von 1,2-Decandiol zu offenbaren, oder befasst sich mit antimikrobieller Wirkung anderer 1,2-Alkandiole, ohne dabei 1,2-Decandiol bzw. die Eigenschaft die Reduktion der Sebumkonzentration der Haut zu beschreiben. Hingewiesen sei insoweit auf die folgenden Dokumente: FR 2,755,371; JP 11 310506; WO 99/11237; WO 99/56715; WO 99/56716; JP 20 0044419; JP 11 335258; JP 10 053510; J. Food Sc. 42(3), 699-701; EP 1 000 542; DE 199 24 496 und JP 20 0148720.

In US 6,123,953 wird dargestellt, dass unverzweigte 1,2-Alkandiole mit einer Kettenlänge von insgesamt 5 bis 14 C-Atomen und vorzugsweise 6 bis 8 C-Atomen als Mittel mit antimikrobieller Aktivität zur topischen Applikation auf der Haut und hierbei insbesondere zur Behandlung von Akne, zur Behandlung von Kapillarschichten, zur Desinfektion kleiner Wunden (Kratzer), zur Desinfektion der Hände von Chirurgen und Kranken sowie zur Desinfektion der Euter von milchgebenden Tieren geeignet sind. 1,2-Decandiol wird nicht explizit genannt. US 6,123,953 offenbart ferner, dass Formulierungen, die neben einem 1,2-Alkandiol ein Gel des Glyceryl-Polymethacrylat-Typs enthalten, in niedrigen Konzentration zur Behandlung von Akne, Hautschwierigkeiten, Impetigo, Mikroorganismen basierten Körpergerüchen, ,,athlete's food" und dergleichen eingesetzt werden können. Diese Einsetzbarkeit wird dabei auf eine synergistische Wechselwirkung zwischen dem Gel und dem Alkandiol zurückgeführt. Als bevorzugtes 1,2-Alkandiol zur Behandlung von Akne wird in dieser Schrift 1,2-Octandiol angegeben. Eine Reduktion der Sebumkonzentration in der Haut durch diese 1,2-Alkandiole wird dort nicht offenbart.

Wie oben bereits beschrieben, offenbart EP 1 598 064 A1 die Verwendung von 1,2-Decandiol in Kombination mit alpha- und/oder beta-Hydroxysäuren in Anti-Akne-Produkten, wobei hier nur auf die synergistische Zusammenwirkung dieser Kombination in Bezug auf die antimikrobielle Eigenschaft auf *P. acnes* abgestellt wird. Eine Reduktion der Sebumkonzentration der Haut wird in EP 1 598 064 A1 allerdings nicht offenbart.

Zusammengefasst bedeutet dies, dass der Stand der Technik lediglich davon ausgeht, dass 1,2-Alkandiole, insbesondere 1,2-Decandiol, antibakterielle Eigenschaften in Bezug auf *Propionibacterium acnes* sowie auf Körpergeruch verursachende Bakterien haben. Demzufolge wird nicht gelehrt, dass 1,2-Decandiol eine Reduktion der Sebumkonzentration der Haut verursacht.

Die bevorzugten Ausgestaltungen der vorliegenden Erfindung werden in den abhängigen Ansprüchen dargestellt und im Folgenden näher beschrieben.

Mittel zur Reduktion der Sebumkonzentration der Haut werden bevorzugt ausgewählt aus der Produktgruppe bestehend aus:
Reinigungsemulsion, Reinigungsmilch, cremige Waschemulsion, reinigendes Waschgel, Reinigungsmilch, klärendes und/oder pflegendes Gesichtswasser, Reinigungstuch sowie Shampoo und/oder Haarkurspülung im Kopfhautbereich. Häufige und bevorzugt in derartigen Produkten enthaltene und besonders geeignete Hilfs- und/oder Zusatzstoffe, bevorzugt ein, zwei, drei, vier, fünf, sechs, sieben acht, neun, zehn oder mehr, weiter bevorzugt drei oder mehr, noch weiter bevorzugt vier oder mehr Hilfs- und/oder Zusatzstoffe werden ausgewählt aus der Gruppe bestehend aus:
   Wasser; Ethanol; Tenside, bevorzugt Natriumlauryl sulfat, Lauryl glucoside und Cocoamidopropyl Betaine; Polyethylenglycol-Derivate, bevorzugt Beispiel PEG-8 und PEG20 (PEG entspricht Polyethylenglycol); Polyethylenglycol-Ester; bevorzugt PEG5 ethylhexanoate; hautfeuchte-regulierende Stoffe, bevorzugt Glycerin, Propylen- und Butylenglycol, Harnstoff und Sorbitol; gut spreitende kosmetische Ester, bevorzugt Ethylhexyl ethylhexanoate, Ethylhexyl isononanoate und Cetearyl ethylhexanoate; sowie in speziellen Anwendungen gegebenfalls hautberuhigende und/oder antiinflammatorisch wirksame Zusätze, bevorzugt alpha-Bisabolol, Panthenol, Allantoin, Anthranilsäureamide - inbesondere die in WO 2004/047833 genannten, bevorzugt Dihydroavenanthramid D - und Pflanzenextrakte, bevorzugt Hamamelis Extrakte, Ingwer Extrakte und Haferextrakte; Talkum und Bentonit.
   Bevorzugt wird das topisch zu applizierende, kosmetische und/oder dermatologische Mittel zur Reduktion der Sebumkonzentration der Haut als Mittel zur Pflege, Prophylaxe und/oder Behandlung von Seborrhoe eingesetzt.
   Die oben angeführten Produktgruppen sowie die bevorzugten Hilfs- und/oder Zusatzstoffe für Mittel zur Reduktion der Sebumkonzentration der Haut sind auch als Mittel zur Pflege, Prophylaxe und/oder Behandlung von Seborrhoe bevorzugt.
   Bevorzugt wird ein Mittel zudem zur Pflege, Prophylaxe und/oder Behandlung von Seborrhoe zur Pflege, Prophylaxe und/oder Behandlung fettig-öliger und/oder unreiner Haut eingesetzt.
   Die oben angeführten Produktgruppen sowie die bevorzugten Hilfs- und/oder Zusatzstoffe für Mittel zur Reduktion der Sebumkonzentration der Haut sind auch zur Pflege, Prophylaxe und/oder Behandlung fettig-öliger und/oder unreiner Haut bevorzugt.
   Da der seborrhoische Zustand der Haut einen idealen Nährboden für Bakterien- und Pilzwachstum und in Folge dessen für die Ausbildung von Akne darstellt, ist ein Mittel zur Prophylaxe und/oder Behandlung von unreiner Haut ebenfalls ein bevorzugtes Mittel zur Prophylaxe und/oder Behandlung von Akne.
   Die oben angeführten Produktgruppen sowie die bevorzugten Hilfs- und/oder Zusatzstoffe für Mittel zur Reduktion der Sebumkonzentration der Haut sind auch als Mittel zur Prophylaxe und/oder Behandlung von Akne bevorzugt.
   Eine bevorzugte topisch zu applizierende, kosmetische Zubereitung umfasst folgende Bestandteile oder besteht aus folgenden Bestandteilen:
      - eine Menge an 1,2-Decandiol die ausreicht, um die Sebumkonzentration der Haut zu reduzieren,
      - ein, zwei, drei, vier, fünf, sechs, sieben, acht, neun, zehn oder mehrere, bevorzugt drei oder mehrere, weiter bevorzugt vier oder mehrere Reinigungshilfsstoffe,
      - gegebenenfalls ein oder mehrere weitere Hilfs- und/oder Zusatzstoffe.
   Eine solche kosmetische Zubereitung ist besonders geeignet für die Reinigung der fettig-öligen und/oder der unreinen Haut.
   Bevorzugt werden ein, zwei, drei, vier, fünf, sechs, sieben, acht, neun, zehn oder mehrere, bevorzugt drei oder mehrere, weiter bevorzugt vier oder mehrere Reinigungshilfsstoffe ausgewählt aus der Gruppe bestehend aus:
      Wasser; Ethanol; Tenside, bevorzugt Natriumlauryl sulfat, Lauryl glucoside und
      Cocoamidopropyl Betaine; Polyethylenglycol-Derivate, bevorzugt PEG-8 und PEG-20; Polyethylenglycol-Ester, bevorzugt PEG-5 ethylhexanoate; hautfeuchte-regulierende Stoffe, bevorzugt Polyole - hiervor bevorzugt Glycerin, Propylen- oder Butylenglycol -, Harnstoff und Sorbitol; gut spreitende kosmetische Ester, bevorzugt Ethylhexyl ethylhexanoate, Ethylhexyl isononanoate und Cetearyl ethylhexanoate; sowie in speziellen Anwendungen gegebenefalls hautberuhigende und/oder antiinflammatorisch wirksame Verbindungen, bevorzugt alpha-Bisabolol, Panthenol, Allantoin, Anthranilsäure-Derivate - insbesondere die in WO 2004/047833 genannten, hiervon bevorzugt Dihydroavenanthramid D - und Pflanzenextrakte - hiervon bevorzugt Hamamelis Extrakte, Ingwer Extrakte oder Hafer Extrakte sowie Talkum und Bentonit.

Eine weitere bevorzugte topisch zu applizierende, kosmetische und/oder dermatologische Zubereitung umfasst die folgenden Bestandteile oder besteht aus folgenden Bestandteilen:
- eine Menge an 1,2-Decandiol, die ausreicht, um die Sebumkonzentration der Haut zu reduzieren,
- ein, zwei, drei, vier oder mehrere Wirkstoffe ausgewählt aus der Gruppe bestehend aus Benzoylperoxid; Salicylsäure; Milchsäure; Azelainsäure; Retinoide, bevorzugt all-trans-Retinsäure, all-trans-Retinal, cis-13-Retinsäure und Adaptalen; Antibiotika, bevorzugt Clindamycin, Erythromycin und Tetracyclin; Schwefel; Chlorhexidin; Triclosan; Farnesol; Phenoxyethanol; Isoflavonoide, bevorzugt Genistein, Daidzein, Genistin und Daizin sowie Antiandrogene, bevorzugt 5alpha-Reduktasehemmer
   und
- gegebenenfalls ein oder mehrere weitere Hilfs- und/oder Zusatzstoffe mit der Maßgabe, dass wenn die Zubereitung eine Konzentration von 1 Gew.-% Milchsäure enthält, die Konzentration von 1,2-Decandiol nicht 0,2 Gew.-% betragen darf, jeweils bezogen auf das Gesamtgewicht der Zubereitung.

In einer weiteren bevorzugten Ausführungsform der topisch zu applizierenden, kosmetischen und/oder dermatologischen Zubereitung werden ein, zwei, drei oder mehrere Wirkstoffe ausgewählt aus der Gruppe bestehend aus Benzoylperoxid, Salicylsäure, Azelainsäure, all-trans-Retinsäure, all-trans-Retinal, cis-13-Retinsäure, Adaptalen, Clindamycin, Erythromycin und Tetracyclin.

Die Menge eines, zweier, dreier oder mehrerer (bevorzugter) Wirkstoffe in den erfindungsgemäßen Zubereitungen beträgt jeweils vorzugsweise 0,0001 bis 20 Gew.-%, besonders bevorzugt 0,0001 - 10 Gew.-%, insbesondere 0,001 - 5 Gew.-%, jeweils bezogen auf das Gesamtgewicht der Zubereitung, wobei die Gesamtmenge aller (bevorzugter) Wirkstoffe in den erfindungsgemäßen Zubereitungen 0,0001 bis 20 Gew.-%, bevorzugt 0,001 bis 10 Gew.-% beträgt.

Da Akne zumeist mit entzündlichen Prozessen einhergeht, können die kosmetischen und/oder dermatologischen erfindungsgemäße Zubereitungen, die 1,2-Decandiol enthalten, besonders vorteilhaft auch ein, zwei, drei oder mehr, bevorzugt ein, zwei oder drei entzündungshemmende und/oder rötungs- und/oder weitere juckreizlindernde Wirkstoffe (Entzündungshemmer) enthalten. Es können hierbei alle für kosmetische und/oder dermatologische Anwendungen geeigneten oder gebräuchlichen entzündungshemmenden bzw. rötungs- und/oder juckreizlindernden Wirkstoffe verwendet werden.

Vorteilhaft werden die entzündungshemmenden bzw. rötungs- und/oder juckreizlindernden Wirkstoff ausgewählt aus der Gruppe der steroidalen entzündungshemmenden Wirkstoffe vom Kortikosteroiden-Typ, besonders bevorzugt Hydrocortison; Hydrocortison-Derivate, bevorzugt Hydrocortison-17-butyrat, Dexamethason, Dexamethasonphosphat, Methylprednisolon oder Cortison, wobei die Auflistung durch Zusatz weiterer steroidaler Entzündungshemmer erweiterbar ist.

Alternativ oder kumulativ können auch nicht steroidale entzündungshemmenden bzw. rötungs- und/oder juckreizlindernden Wirkstoffe bevorzugt eingesetzt werden. Beispielhaft erwähnt werden sollen hier Oxicame, bevorzugt Piroxicam oder Tenoxicam; Salicylate, bevorzugt Aspirin, Disalcid, Solprin oder Fendosal; Essigsäre-Derivate, bevorzugt Diclofenac, Fenclofenac, Indomethacin, Sulindac, Tolmetin, oder Clindanac; Fenamate, bevorzugt Mefenamic, Meclofenamic, Flufenamic oder Niflumic; Propionsäure-Derivate, bevorzugt Ibuprofen, Naproxen, Benoxaprofen oder Pyrazole, bevorzugt Phenylbutazon, Oxyphenylbutazon, Febrazon oder Azapropazon.

Alternativ oder kumulativ können bevorzugt entzündungshemmende bzw. rötungs-und/oder juckreizlindernde Wirkstoffe eingesetzt werden, die natürlich vorkommen. Geeignete Stoffe kommen in Pflanzenextrakten, speziell hochwirksamen Pflanzenextrakt-Fraktionen sowie aus Pflanzenextrakten isolierte hochreine Wirksubstanzen vor, und werden bevorzugt ausgewählt aus Kamille, Aloe vera, Commiphora-Arten, Rubia-Arten, Weiden, Weidenröschen, Hafer, Calendula, Arnika, Johanniskraut, Geißblatt, Rosmarin, Melisse, Ingwer, Passiflora incarnata, Hamamelis, Pueraria, Dianthus oder Echinacea sowie Reinsubstanzen, bevorzugt u.a. Bisabolol, Apigenin, Apigenin-7-glucosid, Rosmarinsäure, Boswelliasäure, Phytosterole, Glycyrrhizinsäure, Glabridin, Licochalkon A, Gingerole und Anthranilsäureamide, bevorzugt insbesondere Avenanthramid oder Dianthramid.

Die 1,2-Decandiol enthaltenden Formulierungen können somit auch Gemische aus zwei oder mehreren, bevorzugt zwei oder drei entzündungshemmenden bzw. rötungs- und/oder juckreizlindernden Wirkstoffen aus der Gruppe der steroidalen entzündungshemmenden Verbindungen, der nicht steroidalen Entzündungshemmer und/oder der natürlichen vorkommenden Entzündungshemmer enthalten.

Die Menge der entzündungshemmenden bzw. rötungs- und/oder juckreizlindernden Wirkstoffe (eine oder mehrere Verbindungen) in den erfindungsgemäßen Zubereitungen beträgt vorzugsweise 0,0001 bis 20 Gew.-%, besonders bevorzugt 0,0001 - 10 Gew.-%, weiter bevorzugt 0,001 - 5 Gew.-%, jeweils bezogen auf das Gesamtgewicht der Zubereitung.

Bevorzugt umfassen die vorgenannten topisch zu applizierenden, kosmetischen und/oder dermatologischen erfindungsgemäßen Zubereitungen ein, zwei, drei, vier, fünf, sechs, sieben, acht, neun, zehn oder mehrere, bevorzugt drei oder mehr, weiter bevorzugt vier oder mehr Reinigungshilfsstoffe ausgewählt aus der Gruppe bestehend aus:
Wasser; Ethanol; Tenside, bevorzugt Natriumlauryl sulfat, Lauryl glucoside und Cocoamidopropyl Betaine; Polyethylenglycol-Derivate, bevorzugt PEG-8 und PEG-20; Polyethylenglycol-Ester, bevorzugt PEG-5 ethylhexanoate; hautfeuchte-regulierende Stoffe, bevorzugt Polyole - hiervor bevorzugt Glycerin Propylen- oder Butylenglycol, Harnstoff und Sorbitol; gut spreitende kosmetische Ester, bevorzugt Ethylhexyl ethylhexanoate, Ethylhexyl isononanoate und Cetearyl ethylhexanoate; sowie in speziellen Anwendungen gegebenefalls hautberuhigende und/oder antiinflammatorisch wirksame Verbindungen, bevorzugt alpha-Bisabolol, Panthenol, Allantoin, Anthranilsäure-Derivate - inbesondere die in WO 2004/047833 genannten, hiervon bevorzugt Dihydroavenanthramid D - und Pflanzenextrakte sowie Talkum und Bentonit.
1,2-Decandiol wird in den vorliegenden topisch zu applizierenden, kosmetischen und/oder dermatologischen erfindungsgemäßen Zubereitungen in einem Gehalt von 0,1 bis 10 Gew.-% und bevorzugt in einer Konzentration von 0,2 bis 5 Gew.-% und ganz besonders bevorzugt in einer Konzentration von 0,5 bis 2 Gew.-%, jeweils bezogen auf das Gesamtgewicht der Zubereitung eingesetzt.
1,2-Decandiol kann dabei in den vorliegenden topisch zu applizierenden, kosmetischen und/oder dermatologischen erfindungsgemäßen Zubereitungen in unterschiedlichster Form zum Einsatz gelangen. So können sie z.B. eine Lösung (z.B. wässrige, wässrig-alkoholische oder alkoholische Lösung, wobei alkoholisch hier nicht für ethanolisch steht), eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O) (jeweils auch in Form von Silikonöl-Emulsionen), eine Hydrodispersion oder Lipodispersion, eine Pickering-Emulsion, einen festen Stift oder auch ein Aerosol darstellen. Auch die Tränkung eines wasserunlöslichen Substrats mit dem erfindungsgemäßen 1,2-Decandiol (z.B. ein Tuch, ein Vlies, ein Pad) kann vorteilhaft sein, wobei es sich bei dieser Applikationsform sowohl um ein trocken anmutendes als auch um ein feucht anmutendes Substrat handeln kann. Weitere vorteilhafte Darreichungsformen des 1,2-Decandiols sind Cremes, Salben, Hydrodispersionen, Lotionen, Tinkturen, Pumpsprays, Aerosolsprays, wässrige Lösungen, Reinigungssubstrate und dergleichen. Die wässrige Phase kann dabei neben Wasser auch andere Inhaltsstoffe enthalten, beispielsweise Diole oder Polyole niedriger C-Zahl, sowie deren Ether, vorzugsweise Propylenglykol, Glycerin, Ethylenglykol, Ethylenglykolmonoethyl- oder -monobutylether, Propylenglykolmonomethyl, - monoethyl- oder -monobutylether, Diethylenglykol- monomethyl oder monoethylether und analoge Produkte, ferner Alkohole niedriger C-Zahl, z.B. Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Zudem können bevorzugte topisch zu applizierende, kosmetische und/oder dermatologische erfindungsgemäße Zubereitungen aus den folgenden Produktgruppen ausgewählt werden:
Reinigungsemulsion, Reinigungsmilch, cremige Waschemulsion, reinigendes Waschgel, Reinigungsmilch, klärendes und/oder pflegendes Gesichtswasser, Reinigungstuch sowie Shampoo und/oder Haarkurspülung im Kopfhautbereich.
1,2-Decandiol lässt sich mit einer Vielzahl weiterer Hilfs- und/oder Zusatzstoffe kombinieren, wobei 1,2-Decandiol zumindest teilweise dazu führen kann, dass Hilfs-und/oder Zusatzstoffe, die eine Wirkung in Hautbereichen mit gesteigerter Sebumproduktion, insbesondere in Talgdrüsenfollikel, aufweisen, das Eindringen dieser Stoffe in diese Gebiete zu unterstützen. Hierdurch ergeben sich bevorzugte kosmetische und/oder dermatologische erfindungsgemäße Zubereitungen bzw. Produkte:
   Kombination mit Hautfeuchte regulierenden Verbindungen:
   1,2-Decandiol lässt sich besonders vorteilhaft zusätzlich mit einem, zwei, drei oder mehreren Hautfeuchte regulierenden Produkten kombinieren. Kosmetische Zubereitungen, die 1,2-Decandiol enthalten, können daher vorteilhafterweise zusätzlich folgende Feuchthalteregulatoren enthalten: Natriumlactat, Harnstoff, Harnstoffderivate, Glycerin, Diole wie Propylenglykol, Hexylenglycol, 1,2-Pentandiol, 1,2-Hexandiol, 1,2-Octandiol oder auch Gemische der genannten Diole wie insbesondere Mischungen aus 1,2-Hexandiol und 1,2-Octandiol, Kollagen, Elastin oder Hyaluronsäure, Diacyladipate, Petrolatum, Urocaninsäure, Lecithin, Panthenol, Phytantriol, Lycopen, (Pseudo-)Ceramide, Glykosphingolipide, Cholesterol, Phytosterole, Chitosan, Chondroitinsulfat, Lanolin, Lanolinester, Aminosäuren, alpha-Hydroxysäuren (z.B. Zitronensäure, Milchsäure, Äpfelsäure) und deren Derivate, Mono-, Di- und Oligosaccharide wie beispielsweise Glucose, Galactose, Fructose, Mannose, Fruchtzucker und Lactose, Polyzucker wie β-Glucane, insbesondere 1,3-1,4-β-Glucan aus Hafer, alpha-Hydroxy-Fettsäuren, Triterpensäuren wie Betulinsäure oder Ursolsäure, Algenextrakte.
   Die Einsatzkonzentration der Feuchthalteregulatoren liegt je nach Substanz im Konzentrationsbereich von 0,1 bis 10 Gew.-% und bevorzugt im Konzentrationsbereich von 0,5 - 5 Gew.-%, jeweils bezogen auf das Gesamtgewicht des anwendungsbereiten kosmetischen und/oder dermatologischen Endprodukts. Diese Angaben gelten insbesondere für vorteilhaft zu verwendende Diole wie Hexylenglycol, 1,2-Pentandiol, 1,2-Hexandiol und 1,2-Octandiol sowie Mischungen aus 1,2-Hexandiol und 1,2-Octandiol.
   Kombination mit Kühlwirkstoffen:
   1,2-Decandiol lässt sich besonders vorteilhaft zusätzlich mit einem, zwei, drei oder mehreren Kühlwirkstoffen kombinieren. Einzelne zur Verwendung im Rahmen der vorliegenden Erfindung bevorzugte Kühlwirkstoffe sind nachfolgend aufgelistet. Der Fachmann kann die nachfolgende Liste um eine Vielzahl weiterer Kühlwirkstoffe ergänzen; die aufgelisteten Kühlwirkstoffe können auch in Kombination miteinander eingesetzt werden: l-Menthol, d-Menthol, racemisches Menthol, Menthonglycerinacetal (Handelsname: Frescolat®MGA), Menthyllactat (Handelsname: Frescolat®ML, vorzugsweise handelt es sich bei Menthyllactat um I-Menthyllactat, insbesondere 1-Menthyl-1-lactat), substituierte Menthyl-3-carbonsäureamide (z.B. Menthyl-3-carbonsäure-N-ethylamid), 2-Isopropyl-N-2,3-trimethylbutanamid, substituierte Cyclohexancarbonsäureamide, 3-Menthoxypropan-1,2-diol, 2-Hydroxyethylmenthylcarbonat, 2-Hydroxypropylmenthylcarbonat, N-Acetylglycinmenthylester, Isopulegol, Menthylhydroxycarbonsäureester (z.B. Menthyl-3-hydroxybutyrat), Monomenthylsuccinat, 2-Mercaptocyclodecanon, Menthyl-2-pyrrolidin-5-oncarboxylat, 2,3-Dihydroxy-p-menthan, 3,3,5-trimethylcyclohexanonglycerinketal, 3-Menthyl-3,6-di- und -trioxaalkanoate, 3-Menthyl methoxyacetat, Icilin.
   Bevorzugte Kühlwirkstoffe sind: l-Menthol, d-Menthol, racemisches Menthol, Menthonglycerinacetal (Handelsname: Frescolat®MGA), Menthyllactat (vorzugsweise l-Menthyllactat, insbesondere I-Menthyl-I-lactat, Handelsname: Frescolat®ML), substituierte Menthyl-3-carbonsäureamide (z.B. Menthyl-3-carbonsäure-N-ethylamid), 2-Isopropyl-N-2,3-trimethylbutanamid, substituierte Cyclohexancarbonsäureamide, 3-Menthoxypropan-1,2-diol, 2-Hydroxyethylmenthylcarbonat, 2-Hydroxypropylmenthylcarbonat, Isopulegol.

Besonders bevorzugte Kühlwirkstoffe sind: 1-Menthol, racemisches Menthol, Menthonglycerinacetal (Handelsname: Frescolat®MGA), Menthyllactat (vorzugsweise 1-Menthyllactat, insbesondere 1-Menthyl-1-lactat, Handelsname:
Frescolat®ML), 3-Menthoxypropan-1,2-diol, 2-Hydroxyethylmenthylcarbonat, 2-Hydroxypropylmenthylcarbonat.

Ganz besonders bevorzugte Kühlwirkstoffe sind: I-Menthol, Menthonglycerinacetal (Handelsname: Frescolat®MGA), Menthyllactat (vorzugsweise I-Menthyllactat, insbesondere 1-Menthyl-1-lactat, Handelsname: Frescolat®ML).

Die Einsatzkonzentration der einzusetzenden Kühlwirkstoffe liegt je nach Substanz vorzugsweise im Konzentrationsbereich von 0,01 bis 20 Gewichtsprozent und bevorzugt im Konzentrationsbereich von 0,1 bis 5 Gewichtsprozent, jeweils bezogen auf das Gesamtgewicht eines anwendungsbereiten kosmetischen oder pharmazeutischen Endprodukts.

### Kombination mit Osmolyten:

Zudem kann 1,2-Decandiol zusammen mit einem, zwei, drei oder mehreren Osmolyten eingesetzt werden. Als Osmolyte seien beispielhaft genannt: Substanzen aus der Gruppe der Zuckeralkohole (myo-Inositol, Mannitol, Sorbitol), quaternäre Amine wie Taurin, Cholin, Betain, Betain-Glycin, Ectoin, Diglycerolphosphat, Phosphorylcholin, Glycerophosphorylcholine, Aminosäuren, bevorzugt Glutamin, Glycin, Alanin, Glutamat, Aspartat oder Prolin, Phosphatidylcholin, Phosphatidylinositol, anorganische Phosphate, sowie Polymere der genannten Verbindungen, bevorzugt Proteine, Peptide, Polyaminosäuren und Polyole. Diese Osmolyte haben zugleich eine hautbefeuchtende Wirkung.

### Kombination mit pflegenden Substanzen:

Zu pflegenden Substanzen, die sich mit 1,2-Decandiol kombinieren lassen, zählen tierische und/oder pflanzliche Fette und Öle wie Olivenöl, Sonnenblumenöl, raffiniertes Sojaöl, Palmöl, Sesamöl, Rapsöl, Mandelöl, Borretschöl, Nachtkerzenöl, Kokosöl, Sheabutter, Jojobaöl, Spermöl, Rindertalg, Klauenöl und Schweineschmalz sowie gegebenfalls weitere pflegende Bestandteile wie zum Beispiel Fettalkohole mit 8-30 C-Atomen. Die verwendeten Fettalkohole können hierbei gesättigt oder ungesättigt bzw. linear oder verzweigt sein.

Zu pflegenden Substanzen, die sich besonders bevorzugt mit 1,2-Decandiol kombinieren lassen, zählen darüber hinaus insbesondere auch
- Ceramide, wobei man unter Ceramiden N-Acylsphingosine (Fettsäureamide des Sphingosins) oder synthetische Analoga solcher Lipide (sogenannte Pseudo-Ceramide) versteht, die das Wasserhaltevermögen des Stratum Corneums deutlich verbessern.
- Phospholipide, beispielsweise Sojalecithin, Ei-Lecithin und Kephaline
- Vaseline, Paraffin- und Silikonöle; zu letzteren zählen unter anderem Dialkyl-und Alkylarylsiloxane wie Dimethylpolysiloxan und Methylphenylpolysiloxan, sowie deren alkoxylierte und quaternierte Derivate

Kombination mit Konservierungsmitteln bzw. Chelatoren:
Kosmetische Zubereitungen, die 1,2-Decandiol enthalten, können auch einen, zwei, drei, vier oder mehrere Wirkstoffe zur Konservierung kosmetischer Produkte sowie schweißhemmende Wirkstoffe (Antitranspirantien) und (Metall)-Chelatoren enthalten.
Vorzugsweise gewählt werden hierbei Konservierungsmittel wie Benzoesäure und ihre Ester und Salze, Propionsäure und ihre Ester und Salze, Salicylsäure, und ihre Ester und Salze, 2,4-Hexadiensäure (Sorbinsäure) und ihre Ester und Salze, Formaldehyd und Paraformaldehyd, 2-Hydroxybiphenylether und seine Salze, 2-Zinksulfidopyridin-N-oxid, anorganische Sulfite und Bisulfite, Natriumjodat, Chlorbutanolum, 4-Ethylquecksilber-(II)5-Amino-1,3-bis(2-Hydroxybenzoesäure, ihre Salze und Ester, Dehydratcetsäure, Ameisensäure, 1,6-Bis(4-amidino-2-bromphenoxy)-n-hexan und seine Salze, das Natriumsalz der Ethylquecksilber-(11)-thiosalicylsäure, Phenylquecksilber und seine Salze, 10-Undecylensäure und ihre Salze, 5-Amino-1,3-bis(2-ethylhexyl)-5-methyl-hexahydropyrimidin, 5-Brom-5-nitro-1,3-dioxan, 2-Brom-2-nitro-1,3-propandiol, 2,4-Dichlorbenzylalkohol, N-(4-Chlorphenyl)-N'-(3,4-dichlorphenyl)-harnstoff, 4-Chlor-m-kresol, 2,4,4'-Trichlor-2'-hydroxy-diphenylether, 4-Chlor-3,5-dimethylphenol, 1,1'-Methylen-bis(3-(1-hydroxymethyl-2,4-dioximidazolidin-5- yl)harnstoff), Poly-(hexamethylendiguanid)-hydrochlorid, 2-Phenoxyethanol, Hexamethylentetramin, 1-(3-Chloroallyl)-3,5,7-triaza-1-azonia-adamantanchlorid, 1(4-Chlorphenoxy)1(1H-imidazol-1-yl)-3,3-dimethyl-2-butanon, 1,3-Bis-(hydroxy-methyl)-5,5-dimethyl-2,4-imidazolidindion, Benzylalkohol, Octopirox, 1,2-Dibrom-2,4-dicyanobutan, 2,2'-Methylen-bis(6-brom-4-chlorphenol), Bromchlorophen, Mischung von 5-Chlor-2-methyl-3(2H)-isothiazolinon und 2-Methyl-3(2H)isothiazlinon mit Magnesiumchlorid und Magnesiumnitrat, 2-Benzyl-4-chlorphenol, 2-Chloracetamid, Chlorhexidin, Chlorhexidinacetat, Chlorhexidingluconat, Chlorhexidinhydrochlorid, 1-Phenoxy-propan-2-ol, N-Alkyl(C₁₂-C₂₂)trimethyl-ammoniumbromid und -chlorid, 4,4-Dimethyl-1,3-oxazo-lidin, N-Hydroxymethyl-N-(1,3-di(hydroxymethyl)-2,5-dioxoimidazolidin-4-yl)-N'-hydroxy-methylharnstoff, 1,6-Bis(4-amidino-phenoxy)-n-hexan und seine Salze, Glutaraldehyd, 5-Ethyl-1-aza-3,7-dioxabicyclo(3.3.0)octan, 3-(4-Chlorphenoxy)-1,2-propandiol, Hyamine, Alkyl-(C₈-C₁₈)-dimethyl-benzylammoniumchlorid, Alkyl-(C₈-C₁₈)-dimethyl-benzylammonium-bromid, Alkyl-(C₈-C₁₈)-dimethyl-benzyl-ammoniumsaccharinat, Benzylhemiformal, 3-Jod-2-propinyl-butylcarbamat, Natrium-hydroxymethyl-aminoacetat oder Natrium-hydroxymethyl-aminoacetat.
Bevorzugt einzusetzende (Metall)-Chelatoren sind hierbei u.a a-Hydroxyfettsäuren, Phytinsäure, Lactoferrin, a-Hydroxysäuren und ihre Salze wie u.a. Zitronensäure oder Äpfelsäure, sowie Galactarsäure, Galacturonsäure, Gluconsäure, Glucuronsäure, Ribonsäure und deren Salze, Huminsäuren, Gallensäuren, Gallenextrakte, Bilirubin, Biliverdin bzw. EDTA, EGTA und deren Derivate.
Kombination mit tierischen und/oder pflanzlichen Proteinhydrolysaten:
1,2-Decandiol kann vorteilhaft auch tierische und/oder pflanzliche Proteinhydrolysate zugesetzt werden. Vorteilhaft sind insoweit insbesondere Elastin, Kollagen-, Keratin-, Milcheiweiß-, Sojaprotein-, Haferprotein-, Erbsenprotein-, Mandelprotein-, und Weizenproteinfraktionen oder entsprechende Proteinhydrolysate aber auch deren Kondensationsprodukte mit Fettsäuren sowie quarternisierte Proteinhydrolysate, wobei die Verwendung pflanzlicher Proteinhydrolysate bevozugt ist.
Kombination mit Lösungsmitteln:
   Sofern eine kosmetische und/oder dermatologische, 1,2-Decandiol enthaltende Zubereitung eine Lösung oder Lotion darstellt, können Lösungsmittel ausgewählt werden aus der Gruppe umfassend oder bestehend aus:
      - Wasser oder wässrige Lösungen;
      - fette Öle, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
      - Alkohole, Diole oder Polyole niedriger C-Zahl, sowie deren Ether, vorzugsweise Isopropanol, Propylenglykol, Glycerin, Ethylenglykol, Ethylenglykolmonoethyl-oder -monobutylether, Propylenglykolmonomethyl, -monoethyl- oder - monobutylether, Diethylenglykolmonomethyl- oder -monoethylether und analoge Produkte.

Bevorzugt können Gemische der vorstehend genannten Lösungsmittel verwendet werden. Hierbei kann Wasser ein weiterer Bestandteil sein.

### Kombination mit Antioxidantien:

Kosmetische Zubereitungen, die 1,2-Decandiol enthalten, können auch Antioxidantien enthalten, wobei alle für kosmetische und/oder dermatologische Anwendungen geeigneten oder gebräuchlichen Antioxidantien verwendet werden können.

### Kombination mit Vitaminen:

Kosmetische Zubereitungen, die 1,2-Decandiol enthalten, können auch Vitamine und Vitaminvorstufen enthalten, wobei alle für kosmetische und/oder dermatologische Anwendungen geeigneten oder gebräuchlichen Vitamine oder Vitaminvorstufen verwendet werden können.

### Kombination mit Hautaufhellern:

1,2-Decandiol kann in zahlreichen Fällen vorteilhaft in Kombination mit hautaufhellenden Wirkstoffen eingesetzt werden. Erfindungsgemäß können hierbei alle für kosmetische und/oder dermatologische Anwendungen geeigneten oder gebräuchlichen hautaufhellenden Wirkstoffe verwendet werden. Vorteilhafte hautaufhellende Wirkstoffe sind insoweit Kojisäure (5-Hydroxy-2-hydroxymethyl-4-pyranon), Kojisäurederivate wie z.B. Kojisäuredipalmitat, Arbutin, Ascorbinsäure, Ascorbinsäurederivate, Hydrochinon, Hydrochinonderivate, Resorcin, schwefelhaltige Moleküle wie z.B. Glutathion oder Cystein, alpha-Hydroxysäuren, (z.B. Zitronensäure, Milchsäure, Apfelsäure) und deren Derivate, N-Acetyl-Tyrosin und - Derivate, Undecenoylphenylalanin, Gluconsäure, 4-Alkylresorcine, Diphenylmethan-Derivate wie z.B. 4-(1-Phenylethyl)1,3-benzenediol Chromon-Derivate wie Aloesin, Flavonoide, Thymolderivate, 1-Aminoethylphosphinsäure, Thioharnstoffderivate, Ellagsäure, Nicotinamid, Zinksalze wie z.B. Zinkchlorid oder -gluconat, Thujaplicin und -Derivate, Triterpene wie Maslinsäure, Sterole wie Ergosterol, Benzofuranone wie Senkyunolid, Vinyl- und Ethylgujacol, Inhibitoren der Stickstoffoxid-Synthese wie z.B., L-Nitroarginin und dessen Derivate, 2,7-Dinitroindazol oder Thiocitrullin, Metallchelatoren (z.B. α-Hydroxyfettsäuren, Palmitinsäure, Phytinsäure, Lactoferrin, Huminsäure, Gallensäure, Gallenextrakte, Bilirubin, Biliverdin, EDTA, EGTA und deren Derivate), Retinoide, Sojamilch, Serin-Protease-Inhibitoren oder Liponsäure oder andere synthetische oder natürliche Wirkstoffe zur Haut- und Haaraufhellung, wobei letztere auch in Form eines Extrakts aus Pflanzen, wie z.B. Bearberry-Extrakt, Reis-Extrakt, Süßholzwurzel-Extrakt oder daraus angereicherte Bestandteile wie Glabridin oder Licochalkon A, Artocarpus-Extrakt, Extrakt von Rumex- und Ramulus-Arten, Extrakte aus Kieferarten (Pinus) und Extrakte aus Vitis-Arten oder daraus angereicherte Stilbenderivate, Extrakt aus Saxifraga, Maulbeer, Scutelleria oder/und Trauben verwendet werden.

### Kombination mit Hautbräunern:

Kosmetische Zubereitungen, die 1,2-Decandiol enthalten, können auch Wirkstoffe mit hautbräunender Wirkung enthalten. Es können insoweit alle für kosmetische und/oder dermatologische Anwendungen geeigneten oder gebräuchlichen hautbräunenden Wirkstoffe verwendet werden. Beispielhaft sei hier das Dihydroxyaceton (DHA; 1,3-Dihydroxy-2-propanon) erwähnt. DHA kann sowohl in monomerer als auch in dimerer Form vorliegen, wobei in kristalliner Form der Anteil an Dimeren überwiegt.

### Kombination mit Sacchariden:

Kosmetische Zubereitungen, die 1,2-Decandiol enthalten, können auch Mono- Di-und Oligosaccharide wie beispielsweise Glucose, Galactose, Fructose, Mannose, Fruchtzucker und Lactose enthalten.

### Kombination mit Pflanzenextrakten:

Kosmetische Zubereitungen, die 1,2-Decandiol enthalten, können auch Pflanzenextrakte enthalten, die üblicherweise durch Extraktion der gesamten Pflanze, in einzelnen Fällen aber auch ausschließlich aus Blüten und/oder Blättern, Holz, Rinde oder Wurzeln der Pflanze hergestellt werden.

### Kombination mit Tensiden:

Kosmetische Zubereitungen, die 1,2-Decandiol enthalten, können, insbesondere wenn kristalline oder mikrokristalline Festkörper, beispielsweise anorganische Mikropigmente in die Zubereitungen eingearbeitet werden sollen, auch anionische, kationische, nichtionische und/oder amphotere Tenside enthalten. Tenside sind amphiphile Stoffe, die organische, unpolare Substanzen in Wasser lösen können. Bei den hydrophilen Anteilen eines Tensidmoleküls handelt es sich dabei meist um polare funktionelle Gruppen, beispielweise -COO-, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch. Typisch für nicht-ionische Tenside sind Polyether-Ketten. Nicht-ionische Tenside bilden in wässrigem Medium keine Ionen.
A. Anionische Tenside

Vorteilhaft zu verwendende anionische Tenside sind Acylaminosäuren (und deren Salze), wie
- Acylglutamate, beispielsweise Natriumacylglutamat, Di-TEA-palmitoyl-aspartat und Natrium Capryl/ Capringlutamat,
- Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
- Sarcosinate, beispielsweise Myristoyl Sarcosin, TEA-lauroylsarcosinat, Natriumlauroylsarcosinat und Natriumcocoylsarkosinat,
- Taurate, beispielsweise Natriumlauroyltaurat und Natriummethylcocoyltaurat,
- Acyllactylate, Lauroyllactylat, Caproyllactylat, Stearoyllactylat
- Alaninate
Carbonsäuren und Derivate, wie
beispielsweise Laurinsäure, Aluminiumstearat, Magnesiumalkanolat und Zinkundecylenat,
- Ester-Carbonsäuren, beispielsweise Calciumstearoyllactylat, Laureth-6 Citrat und Natrium PEG-4 Lauramidcarboxylat,
- Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,

Sulfonsäuren und Salze, wie
- Acyl-isothionate, z.B. Natrium-/ Ammoniumcocoyl-isethionat,
- Alkylarylsulfonate,
- Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
- Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie

Schwefelsäureester, wie
- Alkylethersulfat, beispielsweise Natrium-, Ammonium-, Magnesium-, MIPA, TIPA-Laurethsulfat, Natriummyrethsulfat und Natrium C12-13 Parethsulfat,
- Alkylsulfate, beispielsweise Natrium-, Ammonium- und TEA- Laurylsulfat.

B. Kationische Tenside
Vorteilhaft zu verwendende kationische Tenside sind
- Alkylamine,
- Alkylimidazole,
- Ethoxylierte Amine und
- Quaternäre Tenside.
RNH₂CH₂CH₂COO⁻ (bei pH=7)
RNHCH₂CH₂COO- B⁺ (bei pH=12) B⁺ = beliebiges Kation, z.B. Na⁺

### - Esterquats

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkylamidopropylhydroxysulfain. Die verwendeten kationischen Tenside können ferner bevorzugt gewählt werden aus der Gruppe der quaternären Ammoniumverbindungen, insbesondere Benzyltrialkyl-ammoniumchloride oder - bromide, wie beispielsweise Benzyldimethylstearyl-ammoniumchlorid, ferner Alkyltrialkylammoniumsalze, beispielsweise Cetyltrimethylammoniumchlorid oder - bromid, Alkyldimethylhydroxy-ethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
- Acyl-/dialkylethylendiamin, beispielsweise Natriumacylamphoacetat, Dinatrium-acylamphodipropionat, Dinatriumalkylamphodiacetat, Natriumacylamphohydroxy-propylsulfonat, Dinatriumacylamphodiacetat und Natriumacylamphopropionat,
- N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
- Alkohole,
- Alkanolamide, wie Cocamide MEA/ DEA/ MIPA,
- Aminoxide, wie Cocoamidopropylaminoxid,
- Ester, die durch Veresterung von Carbonsäuren mit Ethylenoxid, Glycerin, Sorbitan oder anderen Alkoholen entstehen,
- Ether, beispielsweise ethoxylierte/propoxylierte Alkohole, ethoxylierte/propoxylierte Ester, ethoxylierte/ propoxylierte Glycerinester, ethoxylierte/propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
- Sucroseester, -Ether
- Polyglycerinester, Diglycerinester, Monoglycerinester
- Methylglucoseester, Ester von Hydroxysäuren

Vorteilhaft ist ferner die Verwendung einer Kombination von anionischen und/oder amphoteren Tensiden mit einem oder mehreren nicht-ionischen Tensiden.

Die oberflächenaktive Substanz kann in einer Konzentration zwischen 1 und 98 Gew.-% in den 1,2-Decandiol enthaltenden Zubereitungen vorliegen, bezogen auf das Gesamtgewicht der Zubereitungen.

### Emulsionen umfassend eine erfindungsgemäße Mischung:

Kosmetische oder dermatologische Zubereitungen, die 1,2-Decandiol enthalten, können auch als Emulsionen vorliegen.

Die Ölphase kann vorteilhaft gewählt werden aus folgender Substanzgruppe:
- Mineralöle, Mineralwachse
- fette Öle, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Vorteilhaft einsetzbar sind nicht komedogen aktive Ester aus gesättigten und/oder ungesättigten verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen. Bevorzugte Esteröle sind 3,5,5-Trimethylhexyl-3,5,5-trimethylhexanoat, 2-Ethylhexylisononanoat, 2-Ethylhexyl-3,5,5-trimethylhexanoat, 2-Ethylhexyl-2-ethylhexanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl vorteilhaft mit 1,2-Decandiol kombiniert werden.

Daneben können aber auch Ester aus aromatischen Carbonsäuren und gesättigten und/oder ungesättigten, verzweigten und/oder unverzweigten Alkoholen einer Kettenlänge von 3 bis 30 C-Atomen.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr. Auch beliebige Abmischungen solcher Öl- und Wachskomponenten sind vorteilhaft einsetzbar. In manchen Fällen ist es auch vorteilhaft, Wachse, beispielsweise Cetylpalmitat, als alleinige Lipidkomponente der Ölphase einzusetzen, vorteilhaft wird die Ölphase gewählt aus der Gruppe , die besteht aus 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid und Dicaprylylether. Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat. Auch die Kohlenwasserstoffe Paraffinöl, Squalan und Squalen lassen sich vorteilhaft verwenden. Vorteilhaft kann die Ölphase ferner einen Gehalt an cyclischen oder linearen Silikonölen aufweisen oder vollständig aus solchen Ölen bestehen, wobei es allerdings bevorzugt ist, außer dem Silikonöl oder den Silikonölen einen zusätzlichen Gehalt an anderen Ölphasenkomponenten zu verwenden. Cyclomethicon (z.B. Decamethylcyclopentasiloxan) kann vorteilhaft als Silikonöl eingesetzt werden. Aber auch andere Silikonöle sind vorteilhaft verwendbar, beispielsweise Undecamethylcyclotrisiloxan, Polydimethylsiloxan und Poly(methyl-phenylsiloxan). Besonders vorteilhaft sind ferner Mischungen aus Cyclomethicon und Isotridecylisononanoat sowie aus Cyclomethicon und 2-Ethylhexylisostearat.

Die wässrige Phase von als Emulsion vorliegenden Zubereitungen, die 1,2-Decandiol enthalten, kann vorteilhafterweise umfassen: Alkohole, Diole oder Polyole niedriger C-Zahl, sowie deren Ether, vorzugsweise Ethanol, Isopropanol, Propylenglykol, Glycerin, Ethylenglykol, Ethylenglykol- monoethyl- oder - monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropyl-methylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Als Emulsion vorliegende Zubereitungen, die 1,2-Decandiol enthalten, umfassen vorteilhaft einen oder mehrere Emulgatoren. O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel R-COO-(-CH₂-CH₂-O-)ₙ-H,
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

   R-COO-(-CH₂-CH₂-O-)ₙ-OOH, und n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel R-O-(-CH₂-CH(CH₃)-O-)ₙ-H
- der Polypropylenglycolether der allgemeinen Formel

   R-O-(-CH₂-CH(CH₃)-O-)ₙ-R'
- der propoxylierten Wollwachsalkohole,
- der veretherten Fettsäurepropoxylate R-COO-(-CH₂-CH(CH₃)-O-)ₙ-R'
- der veresterten Fettsäurepropoxylate der allgemeinen Formel

   R-COO-(-CH₂-CH(CH₃)-O-)ₙ-C(O)-R'
- der Fettsäurepropoxylate der allgemeinen Formel

   R-COO-(-CH₂-CH(CH₃)-O-)n-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)-O-)ₙ-CH₂-COOH,
- der Alkylethersulfate bzw. die diesen Sulfaten zugrundeliegenden Säuren
   der allgemeinen Formel R-O-(-CH₂-CH(CH₃)-O-)ₙ-SO₃-H,
- der Fettalkoholethoxylate/propoxylate der allgemeinen Formel R-O-Xₙ-Yₘ-H
- der Polypropylenglycolether der allgemeinen Formel R-O-Xₙ-Yₘ-R'
- der veretherten Fettsäurepropoxylate der allgemeinen Formel R-COO-Xₙ-Yₘ-R'
- der Fettsäureethoxylate/propoxylate der allgemeinen Formel R-COO-Xₙ-Yₘ-H.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen. Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(13)stearylether (Steareth-13), Polyethylenglycol(14)stearylether (Steareth-14), Polyethylenglycol(15)stearylether (Steareth-15), Polyethylenglycol(1 6)stearylether (Steareth-16), Polyethylenglycol(17)stearylether (Steareth-17), Polyethylenglycol-(18)stearylether (Steareth-18), Polyethylenglycol(19)stearylether (Steareth-19), Polyethylenglycol(20)stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-(18)isostearylether (Isosteareth-18), Polyethylenglycol(19)isostearylether (Isosteareth-19), Polyethylenglycol(20)isostearyl-ether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(1 7)cetylether (Ceteth-17), Polyethylenglycol(18)cetylether (Ceteth-18), Polyethylenglycol(19)cetylether (Ceteth-19), Polyethylenglycol-(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)iso-cetylether (Isoceteth-17), Polyethylenglycol(18)isocetylether (Isoceteth-18), Polyethylenglycol(19)isocetylether(Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(1 2)laurylether (Laureth-12), Polyethylenglycol(12)isolaurylether (Isolaureth12), Polyethylenglycol(1 3)cetylstearylether (Ceteareth-13), Polyethylenglycol(1 4)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol-(18)cetylstearylether (Ceteareth-18), Polyethylenglycol-(19)cetylstearylether (Ceteareth-19), Polyethylenglycol(20)cetylstearylether (Ceteareth-20).

Es ist ferner von Vorteil, die Fettsäureethoxylate aus folgender Gruppe zu wählen:

Polyethylenglycol(20)stearat, Polyethylenglycol(21)stearat, Polyethylenglycol-(22)stearat, Polyethylenglycol(23)stearat, Polyethylenglycol(24)stearat, Polyethylenglycol(25)stearat, Polyethylenglycol(12)isostearat, Polyethylenglycol-(13)isostearat, Polyethylenglycol-(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol-(22)isostearat, Polyethylenglycol(23)isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth 1-4 sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprylat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 bis 18 C-Atomen, Diglycerinester gesättigter und/oder ungesättigter, verzweigter und/oder unverzweigter Alkancarbonsäuren einer Kettenlänge von 8 bis 24, insbesondere 12 bis 18 C-Atomen, Monoglycerinether gesättigter und/oder ungesättigter, verzweigter und/oder unverzweigter Alkohole einer Kettenlänge von 8 bis 24, insbesondere 12 bis 18 C-Atomen, Diglycerinether gesättigter und/oder ungesättigter, verzweigter und/oder unverzweigter Alkohole einer Kettenlänge von 8 bis 24, insbesondere 12 bis 18 C-Atomen, Propylenglycolester gesättigter und/oder ungesättigter, verzweigter und/oder unverzweigter Alkancarbonsäuren einer Kettenlänge von 8 bis 24, insbesondere 12 bis 18 C-Atomen sowie Sorbitanester gesättigter und/oder ungesättigter, verzweigter und/oder unverzweigter Alkancarbonsäuren einer Kettenlänge von 8 bis 24, insbesondere 12 bis 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether(Steareth-2), Glycerylmonolaurat, Glycerylmonocaprinat, Glycerylmonocaprylat.

### Kombination mit Sonnenschutzmitteln:

Zur Anwendung werden die 1,2-Decandiol enthaltenden kosmetischen und/oder dermatologischen/keratologischen Formulierungen in der für Kosmetika und Dermatika üblichen Weise auf die Haut in ausreichender Menge aufgebracht. Besondere Vorteile bieten dabei auch kosmetische und dermatologische Zubereitungen, die 1,2-Decandiol enthalten und zusätzlich als Sonnenschutzmittel wirken. Vorteilhaft enthalten diese Zubereitungen mindestens einen UVA-Filter und/oder mindestens einen UVB-Filter und/oder mindestens ein anorganisches Pigment. Die Zubereitungen können dabei in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

### Kombination mit Verdickern

1,2-Decandiol kann vorteilhaft auch mit Verdickern kombiniert werden Geeignete Verdicker sind: Homopolymere der Acrylsäure mit einem Molekulargewicht von 2000000 bis 6000000 wie z.B. Handelsprodukt Carbopole. Weiter Verdicker werden vertrieben unter den Namen Carbopol 940, Carbopol EDTA 2001 oder Modarez V 600 PX. Polymere aus Acrylsäure und Acrylamid (Natriumsalz) mit einem Molekulargewicht von 2000000 bis 6000000 wie z.B. Hostacerin PN 73 oder das unter dem Namen Amigel vertriebene Sclerotium Gum. Ausserdem geeignet sind Copolymere der Acrylsäure oder der Methacrylsäure wie z.B. Carbopol 1342 oder Permulen TRI. Weitere Verdickertypen sind Polyglycole, Cellulosederivate, insbesondere Hydroxyalkylcellulosen sowie Alginate, Carageenan und anorganische Verdicker wie z.B. natürliche oder synthetische Bentonite.

### Kombination mit Füllstoffen:

1,2-Decandiol kann ferner vorteilhaft, wenngleich nicht zwingend, mit Füllstoffen kombiniert werden, welche z.B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z.B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z.B. Bornitrid etc.) und/oder Aerosile<(R)> (CAS-Nr. 7631-86-9).

### Kombination mit kosmetischen Hilfsstoffen:

1,2-Decandiol kann in kosmetischen Zubereitungen vorteilhaft auch mit kosmetischen Hilfsstoffen kombiniert werden, wie sie üblicherweise in solchen Zubereitungen verwendet werden, z.B. Antioxidantien, Parfümöle, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, weitere anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate. Erfindungsgemäß können hierbei alle denkbaren für kosmetische und/oder dermatologische Anwendungen geeigneten oder gebräuchlichen Antioxidantien, Parfümöle, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse, Alkohole, Polyole, Polymere, Schaumstabilisatoren, Elektrolyte, organische Lösungsmittel oder Silikonderivate verwendet werden.

### Kombination mit Riechstoffen:

1,2-Decandiol kann auch als Bestandteil von Duftstoffkompositionen (Riechstoffkompositionen) für Haar- und Kopfhautpflegeprodukte eingesetzt werden und insbesondere aufgrund seiner spezifischen Wirksamkeit beispielsweise einem parfümierten Fertigprodukt eine zusätzliche Sebum-reduzierende und Anti-Akne Wirkung verleihen. Besonders bevorzugte Duftstoffkompositionen umfassen (a) eine sensorisch wirksame Menge eines Riechstoffes oder der Summe aus zwei, drei odher mehr Riechstoffen, (b) eine Sebum-reduzierende Wirkung verleihende Menge des 1,2-Decandiols sowie (c) ggf. einen oder mehrere Trägerstoffe und/oder Zusatzstoffe. Da der Anteil an Parfüm in einem kosmetischen Fertigprodukt häufig im Bereich von ca. 1 Gew.-% liegt, wird ein Parfüm, welches 1,2-Decandiol enthält, vorzugsweise zu etwa 0,1-10 Gew.-% aus 1,2-Decandiol bestehen. Als besonders vorteilhaft hat es sich erwiesen, dass 1,2-Decandiol nur einen schwachen Eigengeruch besitzen oder gar völlig geruchlos ist; denn diese Eigenschaft prädestiniert sie insbesondere für den Einsatz in einer Duftstoffkomposition.

### Kombination mit Farbstoffen und/oder Farbpigmenten:

Kosmetische und dermatologische Zubereitungen die 1,2-Decandiol enthalten können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisengxide (z.B. Fe2O3, Fe3O4, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus dem Rowe Colour Index, 3. Auflage, Society of Dyers and Colourists, Bradford, England, 1971 zu wählen. Sofern die 1,2-Decandiol enthaltenden Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z.B. Paprikaextrakte, β-Carotin oder Cochenille. Vorteilhaft sind ferner Formulierungen mit einem Gehalt an Perlglanzpigmenten.

1,2-Decandiol lässt sich dabei ohne Schwierigkeiten in gängige kosmetische oder dermatologische/keratologische erfindungsgemäße Zubereitungen wie u.a. Pumpsprays, Aerosolsprays, Cremes, Salben, Tinkturen, Lotionen, Reinigungsemulsionen, Reinigungsmilch, cremige Waschemulsionen, reinigende Waschgele, reinigende und/oder pflegende Gesichtswässer, Reinigungstücher sowie im Bereich Kopfhaut auch in Shampoos, Haarkurbasen und dergleichen einarbeiten. Hierbei ist es auch möglich und in manchen Fällen vorteilhaft, 1,2-Decandiol mit weiteren Wirkstoffen zu kombinieren. Die 1,2-Decandiol enthaltenden kosmetischen und/oder dermatologischen/keratologischen erfindungsgemäßen Zubereitungen können hierbei ansonsten wie üblich zusammengesetzt sein und zur Behandlung der Haut im Sinne einer dermatologischen/keratologischen Behandlung oder einer Behandlung im Sinne der pflegenden Kosmetik dienen. 1,2-Decandiol kann darüber hinaus aber auch in Schminkprodukten in der dekorativen Kosmetik eingesetzt werden.

Weitere bevorzugte Ausgestaltungen der erfindungsgemäßen Mischungen sowie der erfindungsgemäßen Verfahren und Verwendungen lassen sich den nachfolgenden Beispielen und den zugehörigen Tabellen entnehmen:

### Ausführungsbeispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1

### Tropfpunkterniedrigung eines Modellsebums enthaltend 1,2-Decandiol

Um zu prüfen, inwieweit 1,2-Decandiol Sebum-fluidisierende Eigenschaft besitzt und somit auf physikalischem Wege die Sebum-Konzentration auf der Hautoberfläche und insbesondere in den Talgdrüsenfollikeln zu reduzieren vermag, wurde zunächst der Einfluss von 1,2-Decandiol auf den Tropfpunkt eines Modellsebums untersucht.

Zur Herstellung des Modellsebums wurden folgende in der Literatur beschriebenen Untersuchungen zur Zusammensetzung von menschlichem Sebum herangezogen:
(A) "Differential scanning calorimetry studies of sebum models", J.Cosmet.Sci., 52, 211-224 Average composition of human skin surface lipids for 17 subjects);
(B)"Das seborrhoische Ekzem-Ein Spannungsfeld in der Dermatologie" Uniklinik Münster Prof. Dr. med T.A. Luger, Dissertation Frau Kathrin Decker 2004;
(C) "The role of sebaceous gland activity and scalp microfloral metabolism in the etiology of seborrhoic dermatitis and dandruff", P&G, Beauty Care Technology Division + Department of Dermatology Chung Ang University, Seoul Korea

Basierend auf diesen Veröffentlichungen wurde ein Modellsebum entwickelt, welches im wesentlichen gesättigte und ungesättigte freie Fettsäuren, wachsartige Substanzen, Triglyceride und Cholesterol enthält und im Detail aus den folgenden Substanzen zusammengesetzt ist: Palmitinsäure, Ölsäure, Stearinsäure, Squalen, Myristyl Myristate, Cetyl Palmitate, Tripalmitin, Triolein, und Cholesterin. Die exakte quantitive Zusammensetzung des Modellsebums (Anteile der Einzelstoffe in Gewichtsprozent; Gew.-%) ist der nachfolgenden Tabelle 1 zu entnehmen.

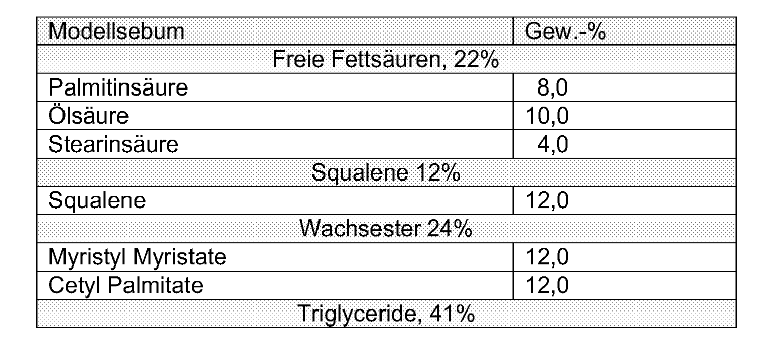

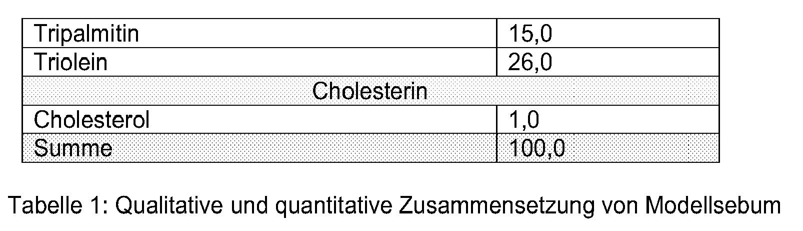

Zur Überprüfung des Einflusses von 1,2-Decandiol auf die physikalischen Eigenschaften des Modellsebums wurden 20 Gew.-% 1,2-Decandiol dem Modellsebum zugesetzt. Anschließend wurden die Tropfpunkte des 1,2-Decandiolfreien Modellsebums und der Modellsebum-Probe enthaltend 20 Gew.-% 1,2-Decandiol mit der Methode zur Tropfpunktbestimmung gemäß AOCS Cc 18-80/ASTM D 3954 ermittelt.

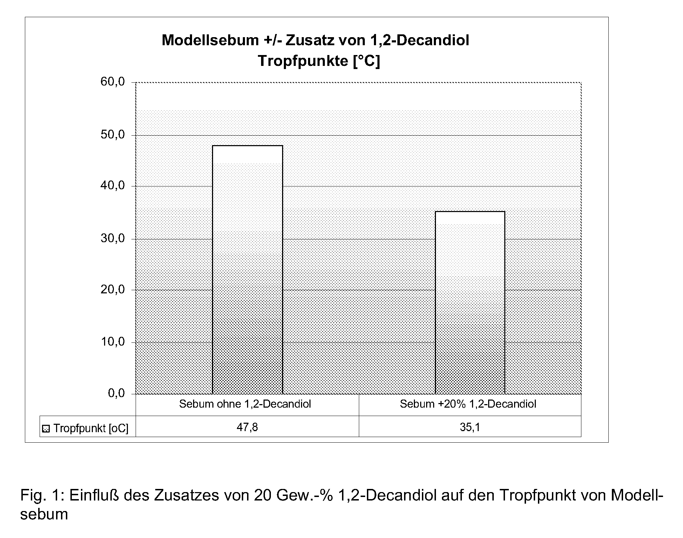

Die Ergebnisse der Untersuchungen sind in Figur 1 graphisch dargestellt.

Die Untersuchungen zeigen eindeutig, dass der Tropfpunkt von Modellsebum durch den Zusatz von 20 Gew.-% 1,2-Decandiol signifikant um 12,7 °C reduziert wird. Somit wird belegt, dass 1,2-Decandiol zur Verflüssigung von Sebum beiträgt.

### Beispiel 2

### Humane in vivo Studie zum Nachweis der Reduktion der Sebumkonzentration der Haut durch eine kosmetische Formulierung enthaltend 2% 1,2-Decandiol

Um zu prüfen, ob die im *in vitro* Experiment ermittelte Reduktion des Tropfpunktes eines Modellsebums bzw. die damit verbundene erhöhte Sebum-Fluidisierung eine klinische Relevanz besitzt und durch diesen spezifischen physikalischen Effekt auch in einer humanen *in vivo* Situation die Sebumkonzentration auf der Hautoberfläche vermindert werden kann, wurde eine klinische Studie an männlichen Probanden mit fettig-öliger und/oder unreiner Haut durchgeführt.

Die experimentellen Parameter sind nachfolgend im Detail beschrieben:
- Probandenanzahl: 20 männliche Personen im Alter von 17 - 19 Jahren
- Testfläche: Stirn (Halbseitentest - Placebo gegen Wirkstoffformulierung)
- Testdauer: 28 Tage
- Testprodukte:
   GS06016-1 (Placeboformulierung ohne 1,2-Decandiol)
   GS06016-2 (Formulierung enthaltend 2% 1,2-Decandiol)

### Gel Formulierungen :

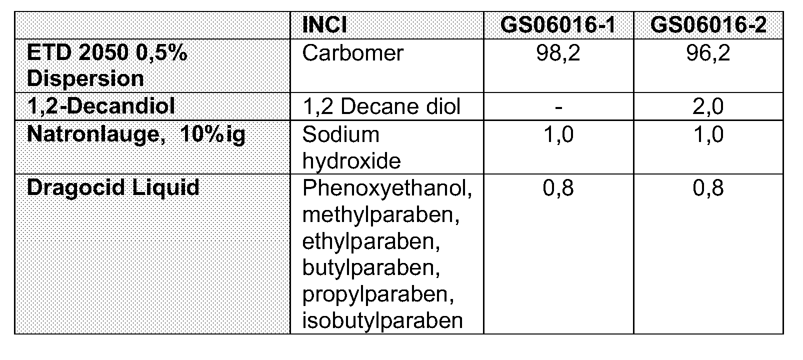

- Anwendungshäufigkeit: 2 mal täglich (morgens und abends) gemäß üblicher täglicher Pflegepraxis
- Beurteilungskriterien:
   a) Sebumgehalt (Sebumetrie; Sebumeter ®SM 810;Courage & Khazaka, Cologne, Germany).
   b) Hautreinheit/Hautzustand (Anzahl/Intensität von Pickel und Pusteln - visuelle Beurteilung)
- Messpunkte: 0, 14, 21 und 28 Tage

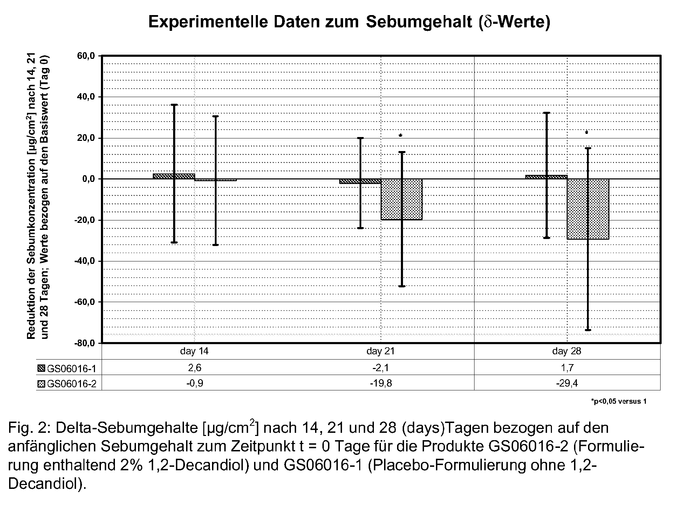

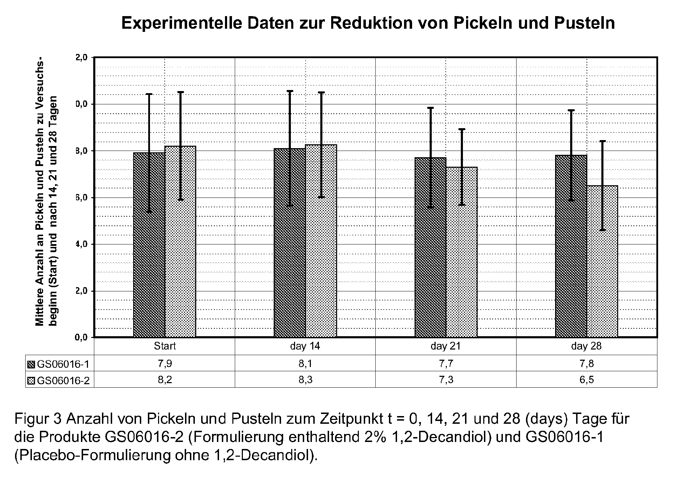

Ergebnis
a) Sebumgehalt (Sebumetrie; Sebumeter ®SM 810;Courage & Khazaka, Köln, Deutschland).
   Wie die humane *in vivo* Studie zeigte, konnte im direkten Vergleich (Halbseitentest/linke gegen rechte Stirnhälfte) bei Anwendung des Produkts GS06016-2 (Formulierung enthaltend 2% 1,2-Decandiol) sowohl nach dem Zeitraum t = 21 Tagen (Reduktion des Sebumgehalts um 19,8 µg/cm² bezogen auf den anfänglichen Sebumgehalt zur Zeit = 0) als auch nach dem Zeitraum t = 28 Tagen (Reduktion des Sebumgehalts um 29,4 µg/cm² bezogen auf den anfänglichen Sebumgehalt zur Zeit t = 0) eine gegenüber GS06016-1 (Placebo-Formulierung ohne 1,2-Decandiol) statistisich signifikante (p < 0,05) Reduktion des Sebumgehalts gemessen werden.
   Für Produkt GS06016-1 (Placebo-Formulierung ohne 1,2-Decandiol) konnte hingegen keine Reduktion des Sebumgehalts nachgewiesen werden. Die Ergebnisse der Sebumreduktion (Delta-Werte jeweils bezogen auf den Sebumgehalt zum Zeitpunkt t = 0 Tage) sind in Figur 2 für die Produkte GS06016-1 und GS06016-2 graphisch dargestellt.
b) Hautreinheit/Hautzustand (Anzahl von Pickel und Pusteln - visuelle Beurteilung)
   Wie die humane *in vivo* Studie ebenfalls zeigte, konnte im direkten Vergleich (Halbseitentest/linke gegen rechte Stirnhälfte) bei Anwendung des Produkts GS06016-2 (Formulierung enthaltend 2% 1,2-Decandiol) nach dem Zeitraum t = 28 Tagen (gemittelte Anzahl an Pickeln und Pusteln: 6,5) eine gegenüber dem Anfangswert (gemittelte Anzahl an Pickeln und Pusteln: 8,2) deutliche Reduktion der Anzahl an Pickeln und Pusteln erzielt werden. Für die Formulierung GS06016-1 (Placebo-Formulierung ohne 1,2-Decandiol) war hingegen keine Reduktion nachweisbar war. Die Ergebnisse sind in Figur 3 graphisch dargestellt.

Diese Ergebnisse zeigen, dass aufgrund des Einsatzes von 1,2-Decandiol eine signifikante Reduktion der Sebumkonzentration der Haut erreicht wird.

### Beispiel 3: Beispielsformulierungen

1= Gesichtswasser gegen fettige, ölige Haut
2= Reinigungsmilch O/W gegen fettige, ölige Haut
3=Anti Akne Make up SPF 8
4=Anti Akne Creme O/W
5=Anti Akne Gel
6=Anti Akne Lotion
7= Waschlotion gegen fettige, ölige Haut
8=Tränkflüssigkeit für Anti Akne Feuchttücher

| Rohstoff | INCI | 1 | 2 | 3 | 4 | 5 | 6 | 7 | 8 |
|---|---|---|---|---|---|---|---|---|---|
| (-)-Alpha-Bisabolol, natürlich(Symrise) | Bisabolol | | | 0,10 | | | | | |
| Abil 350 | Dimethicone | | | | 1,00 | | | | 0,50 |
| Allantoin | Allantoin | 0,10 | 0,20 | | | 0,10 | | | |
| Azelainsäure | Azelainsäure | | | | | | 20,00 | | |
| Benzoylperoxid | Benzoylperoxid | | | | 10,00 | | | | |
| Carbopol EDT 2050 | Carbomer | | | 0,10 | | 1,00 | | | |
| Carbopol Ultrez 21 | Acrylates/C10-30 Alkyl Acrylate Crosspolymer | | | | | | 0,25 | | |
| Citronensäure 10%ig | Citric Acid | | 0,30 | | 0,35 | | | | |
| Clindamycin | | | | | 3,00 | | | | |
| Cremogen M-82 | Water(Aqua), Propylene Glycol, Ethoxydiglycol,Glucose, AesculusHippocastanum (Horse Chestnut) Seed Extract, Salvia Officinalis (Sage)Leaf Extract, Equisetum Arvense Extract, Tussilago Farfara (Coltsfoot)Leaf Extract, Rosmarinus Officinalis(Rosemary)Leaf Extract, UrticaDioica (Nettle) Extract, Chamomilla Recutita (Matricaria) Flower Extract, Melissa Officinalis Leaf Extract | | | | 1,50 | | | | |
| 1,,2Decandiol | 1"2 Decandiol | 0,20 | 1,00 | 2,00 | 0,50 | 0,20 | 0,50 | 1,00 | 2,00 |
| Dow Corning 1503 | Dimethicone, Dimethi conol | | | 2,00 | | | | | |
| Dow Corning 200 Fluid | Dimethicone | | 1,00 | | | | | | |
| Dow Corning 345 Fluid | Cyclomethicone | | | 3,00 | | | 0,50 | | |
| Dracorin 100 (Symrise) | Glyceryl Stearate, PEG-100 Stearate | | 6,00 | | | | | | |
| Dracorin GMS (Symrise) | Glyceryl Stearate | | | | | | 0,50 | | |
| Dracorin GOC (Symrise) | Glyceryl Oleate Citrate, Caprylic/Capric Triglyceride | | | | | | 2,00 | | 2,00 |
| Drago-Beta-Glucan (Symrise) | Water (aqua), Butylene Glycol, Glycerin, Avena Sativa (Oat) Kernel Extract | | | | | | 1,50 | | |
| DragoCalm (Symrise) | Water, Glycerin, Avena Sativa (Oat) Kernel Extract | 0,50 | | | | | | | |
| Dragocide Liquid (Symrise) | Phenoxyethanol, Methylparaben, Ethylparaben, Butylparaben, Propylparaben, Isobutylparaben | | | 0,80 | 0,80 | | | | 0,80 |
| Dragoderm (Symrise) | Glycerin, Triticum Vulgare(Wheat) Gluten, Water(Aqua) | | 2,00 | | | | | | |
| Dragoxat 89 (Symrise) | Ethylhexyl Isononanoate | | | 3,00 | 3,00 | | 5,00 | | |
| EDETA BD | Disodium EDTA | | | 0,10 | | | | | |
| Emulgator Dragil (Symrise) | Glycol Stearate SE | | | | 12,00 | | | | |
| Emulsiphos (Symrise) | Potassium Cetyl Phosphate, Hydrogenated Palm Glycerides | | | 2,00 | | | | | |
| Erythromycin | | | | 3,00 | | | | | |
| Clindamycin | | | | | | | | | |
| Ethanol rein, 96%ig | Alcohol Denat. | 15,00 | 15,00 | | | 15,00 | | | |
| Extrapone Hamamelis (Symrise) | Propylene Glycol, Hamamelis Virginiana (Witch Hazel) Bark/Leaf/TwigExtra ct, Water(Aqua), Alcohol, Hamamelis Virginiana (Witch Hazel) Water, Glucose | 2,00 | | | | | | | |
| Extrapone Apple (Symrise) | Propylene Glycol, Water (Aqua), Pyrus Malus (Apple) Juice | | | | | | | 0,20 | |
| Extrapone Camomile (Symrise) | Water(Aqua), Propylene Glycol, Butylene Glycol, Chamomilla Recutita (Matricaria) Flower Extract, Glucose, Bisabolol | | | | | 1,00 | | | |
| Extrapone Honey GW (Symrise) | Water (Aqua), Glycerin, Honey (Mel) | | | | | | | 0,20 | |
| Extrapone Horsetail (Symrise) | Water(Aqua), Propylene Glycol, Glucose, Equisetum Arvense Extract | 2,00 | | | | | | | |
| Extrapone Pear (Symrise) | Propylene Gylcol, Water (Aqua), Pyrus Communis (Pear) Fruit Juice | | | | | | | 0,20 | |
| Farbe | Colour | | | | | 0,10 | | | |
| Farnesol (Symrise) | Farnesol | 0,20 | | | | 0,30 | 0,30 | | 0,20 |
| Frescolat ML (Symrise) | Menthyl Lactate | | | 1,00 | | | | | |
| Glycerin 85%ig in Wasser | Glycerin | | | 3,00 | | | | | |
| Glycerin | Glycerin | | 2,00 | | | 5,00 | | | 3,00 |
| Hydrolite-5 (1,2-Pentandiol) | Pentylene Glycol | | | | | | 5,00 | 0,50 | |
| Hydroviton -24 (Symrise) | Water (Aqua), Pentylene Glycol, Glycerin, Sodium Lactate, Lactic Acid, Serine, Urea, Sorbitol, Sodium Chloride, Allantoin | | | | | | | | 1,00 |
| Hydroviton (Symrise) | Water (Aqua), Glycerin, Sodium Lactate, Lactic Acid, TEA-Lactate, Serine, Urea, Sorbitol,Sodium Chloride, Allantoin | 1,00 | | | | 1,00 | | | |
| Isoadipat (Symrise) | Diisopropyl Adipate | | | 2,00 | | | | | |
| Isodragol (Symrise) | Triisononanoin | | | | | | 4,00 | | 2,00 |
| Kaliumsorbat | Potassium Sorbate | | | | | | 0,10 | | 0,10 |
| Keltrol RD | Xanthan Gum | | | 0,20 | | | 0,15 | | |
| KSMFST BRAUN C.I.77491,77492, 77499, 77891 | | | | 5,00 | | | | | |
| Lanette 18 | Stearyl Alcohol | | 1,5 | | | | | | |
| Lanette E | Sodium Cetearyl Sulfate | | | 0,80 | | | | | |
| Lanette O | Cetearyl Alcohol | | | 1,20 | | | 1,00 | | |
| LBMFST.TITANDIOXID E171 | Titanium Dioxide (Cl 77891) | | | 3,00 | | | | | |
| Lumorol K 5019 | Disodium Laureth Sulfosuccinate, Sodium Lauryl Sulfoacetate | | | | | | | 25,00 | |
| Menthol | Menthol | 0,05 | | | | | | | |
| Milchsäure 90% | Lacric Acid | | 2,00 | | | | | | |
| Natriumhydroxid 10%ige Lösung | Sodium Hydroxide | | | | | 2,50 | 0,50 | | 0,55 |
| Neo Dragocid Liquid (Symrise) | Triethylene Glycol, Imidazolidinyl Urea, Methylparaben, Propylparaben, Dehydroacetic Acid | | | | | | | 0,50 | |
| Neo Heliopan 303 | Octocrylene | | | 1,5 | | | | | |
| Neo Heliopan 357 (Symrise) | Butyl Methoxydibenzoylmethane | | | 1,20 | | | | | |
| Neo Heliopan APeingesetzt als 22 %ige Lösung neutralisiert mit Triethanolamin, (Symrise) | Disodium Phenyl Dibenzimidazole Tetrasulfonate | | | 1,36 | | | | | |
| Neo Heliopan AV (Symrise) | Ethylhexyl Methoxycinnamate | | | 4,00 | | | | | |
| Neo-PCL ws N (Symrise) | Trideceth-9, PEG-5, Ethylhexanoate, Water (Aqua) | 0,50 | | | | 1,00 | | 1,50 | |
| Neutralöl | Caprylic/Capric Triglyceride | | 5,00 | | | | | | |
| Oxetal VD 92 | Glycereth-90 Isostearate, Laureth-2 | | | | | | | 4,50 | |
| Parfümöl | Parfum | 0,10 | 0,30 | 0,20 | 0,10 | 0,05 | 0,20 | 0,10 | 0,30 |
| PCL -Solid (Symrise) | Stearyl Heptanoate, Stearyl Caprylate | | | | | | 2,50 | | |
| PCL-Liquid 100 (Symrise) | Cetearyl Ethylhexanoate | | 2,00 | | 4,00 | | 5,00 | | 3,00 |
| Pemulen TR-2 | Acrylates/C10-30 Alkyl Acrylate Crosspolymer | | | | | | | | 0,20 |
| Pionier NP 37 N | Sodium Carbomer | | 0,10 | | | | | | |
| Propylenglykol 1,2- | Propylene Glycol | | 3,00 | | 4,00 | | | | |
| Retinal, all-trans | Retinal, all-trans | | | | | | 0,05 | | |
| Retinsäure, all-trans | Tretinoin | | 0,05 | | | | | | |
| Retinsäure, cis 13 | Isotretinoin | | | | 0,05 | | | | |
| Salicylsäure | Salicylic Acid | | | 10,00 | | | | | |
| Solubilizer, Löslichkeitsverbesserer | PEG-40 Hydrogenated Castor Oil, Trideceth-9, Propylene Glycol, Water(aqua) | 0,50 | | | | 1,50 | | 1,00 | |
| SymCalmin (Symrise) | Butylene Glycol, Pentylene Glycol, Hydroxyphenyl PropamidobenzoicAcid | | | | | | | | 1,00 |
| SymDiol 68 (Symrise) | 1,2 Hexanediol, Caprylyl Glycol (1:1 (m/m) | 0,50 | | | | | | | |
| Tetracylcin | | | | | | | 3,00 | | |
| Tego Betain F50 | Cocamidopropyl Betaine | | | | | | | 10,00 | |
| Triethanolamin, 99%ig | Triethanolamine | | | 0,20 | | | | | |
| Wasser | Water (Aqua) | Ad 100 | Ad 100 | Ad 100 | Ad 100 | Ad 100 | Ad 100 | Ad 100 | Ad 100 |
| Witch Hazel- Destillate (Symrise) | Hamamelis Virginiana(Witch Hazel) Water, Water(Aqua), Alcohol | | 0,10 | | | | | | |

## Patentansprüche

1. Verwendung von 1,2-Decandiol zur Reduktion der Sebumkonzentration der Haut.

2. Verwendung von 1,2-Decandiol zur Herstellung eines topisch zu applizierenden kosmetischen und/oder dermatologischen Mittels zur Reduktion der Sebumkonzentration der Haut.

3. Verwendung gemäß Anspruch 2, **dadurch gekennzeichnet, dass** das Mittel zur Behandlung von Seborrhoe geeignet ist.

4. Verwendung gemäß Anspruch 3, **dadurch gekennzeichnet, dass** das Mittel zur Behandlung von Akne geeignet ist.

5. Verwendung gemäß einem der Ansprüche 2 bis 4, **dadurch gekennzeichnet, dass** das Mittel zur Pflege, Prophylaxe und/oder Behandlung von fettig-öliger und/oder unreiner Haut geeignet ist.

6. Topisch zu applizierende kosmetische und/oder dermatologische Zubereitung
**dadurch gekennzeichnet, dass** die Zubereitung folgende Bestandteile umfasst:
- eine Menge an 1,2-Decandiol die ausreicht, um die Sebumkonzentration der Haut zu reduzieren,
- gegebenenfalls ein, zwei, drei, vier, fünf, sechs, sieben, acht, neun, zehn oder mehrere Reinigungshilfsstoffe,
- gegebenenfalls ein, zwei, drei, vier oder mehrere Wirkstoffe ausgewählt aus der Gruppe bestehend aus Benzoylperoxid, Salicylsäure, Milchsäure, Azelainsäure, Retinoide, Antibiotika, Schwefel, Chlorhexidin, Triclosan, Farnesol, Phenoxyethanol, Isoflavonoide und Antiandrogene
und
- gegebenenfalls ein oder mehrere weitere Hilfs- und/oder Zusatzstoffe
mit der Maßgabe, dass wenn die Zubereitung eine Konzentration von 1 Gew.-% Milchsäure enthält, die Konzentration von 1,2-Decandiol nicht 0,2 Gew.-% betragen darf, jeweils bezogen auf die Gesamtzubereitung.

7. Zubereitung gemäß Anspruch 5, **dadurch gekennzeichnet, dass** die Zubereitung ein, zwei, drei, vier, fünf, sechs, sieben, acht, neun, zehn oder mehrere Reinigungshilfsstoffe ausgewählt aus der Gruppe bestehend aus Wasser, Ethanol, Tensiden, Polyethylenglycol-Derivaten, Polyethylenglycol-Ester, hautfeuchte-regulierende Stoffen, Harnstoff, Sorbitol, gut spreitende kosmetische Ester, hautberuhigende und/oder antiinflammatorisch wirksame Verbindungen, Pflanzenextrakte und antiseborrhisch wirksame Stoffe umfasst.

8. Zubereitung gemäß Anspruch 5 oder 6, **dadurch gekennzeichnet, dass** ein, zwei, drei oder mehrere Wirkstoffe ausgewählt werden aus der Gruppe bestehend aus Benzoylperoxid, Salicylsäure, Azelainsäure, all-trans-Retinsäure, all-trans-Retinal, cis-13-Retinsäure, Adaptalen, Clindamycin, Erythromycin und Tetracyclin.

9. Verfahren zur Reduktion der Sebumkonzentration der Haut **dadurch gekennzeichnet, dass** das Verfahren folgende Schritte umfasst:
a) Bereitstellung von 1,2-Decandiol;
b) Herstellung einer Zubereitung gemäß einem der Ansprüche 5 bis 7 und
c) Applikation der nach b) hergestellten Zubereitung auf fettig-ölige und/oder unreine Haut, wobei die Menge an 1,2-Decandiol ausreicht, um die Sebumkonzentration der Haut zu reduzieren.

10. Verwendung von 1,2-Decandiol zur Unterstützung des Eindringens eines, zweier, dreier oder mehrerer Wirkstoffe ausgewählt aus der Gruppe bestehend aus Benzoylperoxid, Salicylsäure, Milchsäure, Azelainsäure, Retinoide, Antibiotika, Schwefel, Chlorhexidin, Triclosan, Farnesol, Phenoxyethanol, Isoflavonoide und Antiandrogene in Hautbereiche mit gesteigerter Sebumproduktion.

11. Verfahren zur Unterstützung des Eindringens eines, zweier, dreier oder mehrerer Wirkstoffe ausgewählt aus der Gruppe bestehend aus Benzoylperoxid, Salicylsäure, Milchsäure, Azelainsäure, Retinoide, Antibiotika, Schwefel, Chlorhexidin, Triclosan, Farnesol, Phenoxyethanol, Isoflavonoide und Antiandrogene in Hautbereiche mit gesteigerter Sebumproduktion, **dadurch gekennzeichnet, dass** das Verfahren folgende Schritte umfasst:
a) Bereitstellung von 1,2-Decandiol,
b) Bereitstellung eines, zweier, dreier oder mehrerer Wirkstoffe ausgewählt aus der Gruppe bestehend aus Benzoylperoxid, Salicylsäure, Milchsäure, Azelainsäure, Retinoide, Antibiotika, Schwefel, Chlorhexidin, Triclosan, Farnesol, Phenoxyethanol, Isoflavonoide, Antiandrogene,
c) Herstellung einer topisch zu applizierenden, kosmetischen und/oder dermatologischen Zubereitung umfassend
- eine Menge an 1,2-Decandiol die ausreicht, um die Sebumkonzentration der Haut zu reduzieren,
- eine wirksame Menge eines, zweier, dreier, vierer oder mehrerer Wirkstoffe ausgewählt aus der Gruppe bestehend aus Benzoylperoxid, Salicylsäure, Milchsäure, Azelainsäure, Retinoide, Antibiotika, Schwefel, Chlorhexidin, Triclosan, Farnesol, Phenoxyethanol, Isoflavonoide und Antiandrogene
- gegebenenfalls ein, zwei, drei, vier, fünf, sechs, sieben, acht, neun, zehn oder mehrere Reinigungshilfsstoffe und
- gegebenenfalls ein oder mehrere weitere Hilfs- und/oder Zusatzstoffe mit der Maßgabe, dass wenn die Zubereitung eine Konzentration von 1 Gew.-% Milchsäure enthält, die Konzentration von 1,2-Decandiol nicht 0,2 Gew.-% betragen darf, jeweils bezogen auf die Gesamtzubereitung und
d) Applikation der nach c) hergestellten Zubereitung auf relevante Hautbereiche.
